# EUROPEAN PATENT APPLICATION

(11) **EP 4 545 561 A1**
(43) Date of publication of application: **30.04.2025**
(21) Application number: 23826272.9
(22) Date of filing: 15.06.2023
(51) Int. Cl.: C07K 16/28, C07K 16/46, C07K 19/00, C12N 5/10, C12N 15/85, A61K 47/68, A61P 35/00, A61P 35/04

(54) **NANOBODY AND NANOBODY-DRUG CONJUGATE TARGETING CD73, METHOD FOR PREPARING SAME, AND USE THEREOF**

(30) Priority: 22.06.2022 CN 202210714822; 04.05.2023 WO PCT/CN2023/092108
(71) Applicant: Fudan University, Shanghai 200433 (CN)
(72) Inventor: YU, Ke, Shanghai 200433 (CN); LIU, Liang, Shanghai 200433 (CN); MENG, Tao, Shanghai 200433 (CN)
(74) Representative: Mewburn Ellis LLP
(86) International application number: PCT/CN2023/100479
(87) International publication number: WO 2023/246623

(57) **Abstract**

The present invention relates to a novel nanobody (Nb) and a nanobody-drug conjugate (NDC) targeting CD73, a method for preparing same, and use thereof. The monoclonal nanobody and the corresponding NDC can efficiently bind to isolated CD73, various tumor cells and CD73 on the surface of an immune cell with high specificity and block the catalytic activity of CD73 enzymes, exhibiting high affinity, low immunogenicity, and a significant anti-tumor effect.

## Description

### TECHNICAL FIELD

The present invention relates to the field of medicine, in particular to a nanobody targeting CD73 (CD73-Nb) and a CD73 nanobody-drug conjugate (CD73-NDC), a method for preparing the same, and use thereof.

### BACKGROUND

In recent years, significant progress has been made in the treatment of tumors, among which targeted therapy and immunotherapy have provided new ideas for tumor treatment. However, great challenges still remain for tumor treatment due to the low response rate and rapid drug resistance of patients to drugs. Therefore, finding new therapeutic targets and pharmaceuticals has become the direction of treating refractory tumors. Recent studies have shown that tumor progression and drug resistance are often related to tumor metastasis, recurrence and immunosuppressive microenvironment, and CD73 is one of the closely related molecular mechanisms.

Abnormal expression of CD73 is found in a variety of tumors, including lung cancer, breast cancer, melanoma, and cerebral glioma, and is closely related to tumor metastasis, recurrence, drug resistance, and immunosuppression. CD73 is a 5'-nucleotidase anchored on the cell membrane surface, and is the main rate-limiting enzyme that dephosphorylates adenosine monophosphate (AMP) to generate adenosine (ADO) through enzymatic reactions. Adenosine participates in a variety of physiological and pathological processes *in vivo* by binding to corresponding adenosine receptors (AlAR, A2AR, A2BR, A3AR), including the formation of an immunosuppressive microenvironment, such as inhibiting the proliferation and function of T lymphocytes, inhibiting the differentiation and maturation of dendritic cells (DC), affecting the polarization of macrophages, and promoting immunosuppressive cells such as myeloid-derived suppressor cells (MDSC) to release anti-inflammatory cytokines such as TGFβ and IL-10, etc. Drug resistance is a major obstacle to the efficacy of tumor treatment. Studies have shown that CD73 is involved in the resistance of tumor chemotherapy drugs, small molecule targeted drugs, and immune checkpoint inhibitors. In addition, CD73 is also involved in the epithelial-mesenchymal transition, angiogenesis, metastasis and recurrence of tumors. Therefore, targeting CD73, either as a single drug or in drug combination, can be provided as a new strategy for tumor treatment.

Monoclonal antibody drugs have brought many improvements to cancer treatment. However, the molecular weight of a traditional monoclonal antibody is 150 kd, which limits its penetration and distribution in solid tumors, therefore the therapeutic effect needs to be further improved. A nanobody (Nb) is an antibody composed only of heavy chains naturally produced by alpacas, wherein the target recognition module consists of a single heavy chain variable region (VHH). A nanobody has a molecular weight of only 12-14 kd, which is expected to overcome the low penetration of traditional monoclonal antibodies in solid tumors. A nanobody can be linked to functional domains such as Fc, other nanobodies, or peptide tags or toxins. Due to its small size, it has higher diffusion rate, vascular permeability, and tumor penetration, and has more uniform tissue distribution than traditional monoclonal antibodies. These characteristics make them particularly suitable for specific and effective tumor targeted therapy *in vivo.* **In** addition, nanobodies also have the advantages of low immunogenicity, high stability, high degradation resistance, low production cost of prokaryotic expression, and easy product characterization. However, despite the many potential advantages, the development of tumor therapeutic drugs based on nanobodies is still in the early exploratory research stage.

Antibody-drug conjugates (ADCs) use the characteristics of monoclonal antibodies to specifically recognize specific antigens on the surface of tumor cells, thereby accurately delivering anti-tumor drugs (such as small molecule chemotherapy drugs, etc.) to tumor target cells, and releasing them to accurately kill tumors. ADCs are currently used for targeted drug delivery to tumor tissues, but due to their large molecular weight and weak intratumor penetration, the drug delivery effect varies greatly in tumors, and their application in solid tumors is not ideal. In addition, ADCs have a long terminal half-life *in vivo,* which may cause unnecessary toxicity of the loaded toxins to normal tissues or cells. A nanobody-drug conjugate (NDCs) prepared from a nanobody not only maintains the advantages of traditional ADCs, but also has high vascular permeability, strong tumor penetration, and fast arrival at target cells. It may increase the accumulation of drugs in tumors, and can also moderately control the plasma exposure and half-life of drugs, which will help further improve the treatment effect and overall therapeutic window for solid tumors, and is expected to become a new class of the most promising anti-tumor drugs.

In summary, based on the important role of CD73 in tumor development, recurrence and drug resistance etc., the development of targeting CD73-Nb and CD73-NDC drugs can provide new strategies of single or combined drug therapy for the clinical treatment of patients with abnormal CD73 expression tumors.

### SUMMARY OF THE INVENTION

The object of the present invention is to provide a nanobody targeting CD73 and a nanobody-drug conjugate, a preparation method and use thereof.

In the first aspect of the present invention, it provides a nanobody targeting CD73, wherein the complementary determining region CDR of the VHH chain in the nanobody is one or more selected from the group consisting of:
(1) CDR1 as shown in SEQ ID NO.1,
   CDR2 as shown in SEQ ID NO.2, and
   CDR3 as shown in SEQ ID NO.3;
   or,
(2) CDR1 as shown in SEQ ID NO.5,
   CDR2 as shown in SEQ ID NO.6, and
   CDR3 as shown in SEQ ID NO.7;
   or,
(3) CDR1 as shown in SEQ ID NO.9,
   CDR2 as shown in SEQ ID NO.10, and
   CDR3 as shown in SEQ ID NO.11,
   or,
(4) CDR1 as shown in SEQ ID NO.13,
   CDR2 as shown in SEQ ID NO.14, and
   CDR3 as shown in SEQ ID NO.15,
   or,
(5) CDR1 as shown in SEQ ID NO.17,
   CDR2 as shown in SEQ ID NO.18, and
   CDR3 as shown in SEQ ID NO.19,
   or,
(6) CDR1 as shown in SEQ ID NO.21,
   CDR2 as shown in SEQ ID NO.22, and
   CDR3 as shown in SEQ ID NO.23,
   or,
(7) CDR1 as shown in SEQ ID NO.25,
   CDR2 as shown in SEQ ID NO.26, and
   CDR3 as shown in SEQ ID NO.27,
   or,
(8) CDR1 as shown in SEQ ID NO.29,
   CDR2 as shown in SEQ ID NO.30, and
   CDR3 as shown in SEQ ID NO.31,
   or,
(9) CDR1 as shown in SEQ ID NO.33,
   CDR2 as shown in SEQ ID NO.34, and
   CDR3 as shown in SEQ ID NO.35; or
(10) CDR1 as shown in SEQ ID NO.5,
   CDR2 as shown in SEQ ID NO.54, and
   CDR3 as shown in SEQ ID NO.7; or
(11) CDR1 as shown in SEQ ID NO.5,
   CDR2 as shown in SEQ ID NO.55, and
   CDR3 as shown in SEQ ID NO.7; or
(12) CDR1 as shown in SEQ ID NO.5,
   CDR2 as shown in SEQ ID NO.56, and
   CDR3 as shown in SEQ ID NO.7.

In another preferred embodiment, any one of the above amino acid sequences further comprises a derivative sequence that is optionally added, deleted, modified and/or substituted with at least one amino acid and may retain the ability to bind to CD73.

In another preferred embodiment, the CDR of the nanobody targeting CD73 comprises a derivative sequence with one amino acid substitutionand may retain the ability to bind to CD73; preferably, N in CDR2 as shown in SEQ ID NO.6 is replaced with an amino acid selected from the group consisting of: A, G, or Q. In another preferred embodiment, the CDR region of VHH chain in the nanobody comprises an amino acid sequence having at least 80%, preferably at least 90%, more preferably at least 95%, and even more preferably at least 99% sequence identity to any one of SEQ ID NOs: 1-36.

In another preferred embodiment, the amino acid sequence of the CDR region of VHH chain in the nanobody comprises one or more amino acid substitutions, preferably conservative amino acid substitutions, compared with any one of SEQ ID NOs: 1-36.

In another preferred embodiment, the VHH chain comprises CDR1, CDR2 and CDR3 selected from the group consisting of:
(1) the complementary determining regions CDR1, CDR2, CDR3 as shown in SEQ ID NO.1, SEQ ID NO.2, SEQ ID NO.3 (corresponding to the CDRs of nanobody 3-D7); or
(2) the complementary determining regions CDR1, CDR2, CDR3 as shown in SEQ ID NO.5, SEQ ID NO.6, SEQ ID NO.7 (corresponding to the CDRs of nanobody 5-E11); or
(3) the complementary determining regions CDR1, CDR2, and CDR3 as shown in SEQ ID NO.9, SEQ ID NO.10, and SEQ ID NO.11 (corresponding to the CDRs of nanobody 4-D04); or
(4) the complementary determining regions CDR1, CDR2, and CDR3 as shown in SEQ ID NO.13, SEQ ID NO.14, and SEQ ID NO.15 (corresponding to the CDRs of nanobody 4-B02); or
(5) the complementary determining regions CDR1, CDR2, and CDR3 as shown in SEQ ID NO.17, SEQ ID NO.18, and SEQ ID NO.19 (corresponding to the CDRs of nanobody 1-E7); or
(6) the complementary determining regions CDR1, CDR2, and CDR3 as shown in SEQ ID NO.21, SEQ ID NO.22, and SEQ ID NO.23 (corresponding to the CDRs of nanobody 2-B 10); or
(7) the complementary determining regions CDR1, CDR2, and CDR3 as shown in SEQ ID NO.25, SEQ ID NO.26, and SEQ ID NO.27 (corresponding to the CDRs of nanobody 2-G9); or
(8) the complementary determining regions CDR1, CDR2, and CDR3 as shown in SEQ ID NO.29, SEQ ID NO.30, and SEQ ID NO.31 (corresponding to the CDRs of nanobody 3-A3); or
(9) the complementary determining regions CDR1, CDR2, and CDR3 as shown in SEQ ID NO.33, SEQ ID NO.34, and SEQ ID NO.35 (corresponding to the CDRs of nanobody 3-F1); or
(10) the complementary determining regions CDR1, CDR2, and CDR3 as shown in SEQ ID NO.5, SEQ ID NO.54, and SEQ ID NO.7 (corresponding to the CDRs of nanobody 5-E11AS or 5-E11ASCS); or
(11) the complementary determining regions CDR1, CDR2, and CDR3 as shown in SEQ ID NO.5, SEQ ID NO.55, and SEQ ID NO.7 (corresponding to the CDRs of nanobody 5-E11GS); or
(12) the complementary determining regions CDR1, CDR2, and CDR3 as shown in SEQ ID NO.5, SEQ ID NO.56, and SEQ ID NO.7 (corresponding to the CDRs of nanobody 5-E11QS). In another preferred embodiment, the CDR1, CDR2, and CDR3 are separated by the framework regions FR1, FR2, FR3, and FR4 of the VHH chain.

In another preferred embodiment, the VHH chain of the nanobody further comprises a framework region (FR).

In another preferred embodiment, the framework region FR is derived from human, mouse, rabbit or camel.

In another preferred embodiment, the framework region FR comprises a human FR region, a mouse FR region or a camel FR region.

In another preferred embodiment, the VHH chain of the nanobody targeting CD73 has an amino acid sequence as shown in SEQ ID NO.4, SEQ ID NO.8, SEQ ID NO.12, SEQ ID NO.16, SEQ ID NO.20, SEQ ID NO.24, SEQ ID NO.28, SEQ ID NO.32 or SEQ ID NO.36. In another preferred embodiment, the VHH chain of the nanobody targeting CD73 has an amino acid sequence as shown in SEQ ID NO.46, SEQ ID NO.47, SEQ ID NO.48, SEQ ID NO.49 (all are point mutation sequences of SEQ ID NO.8).

In another preferred embodiment, the nanobody targeting CD73 is a humanized nanobody having an amino acid sequence as shown in SEQ ID NO.50, SEQ ID NO.51 or SEQ ID NO.52. In another preferred embodiment, the VHH chain of the nanobody targeting CD73 has an amino acid sequence as shown in SEQ ID NO.50, SEQ ID NO.51 or SEQ ID NO.52 of the 3-D7 humanized nanobody.

In the second aspect of the present invention, it provides an antibody targeting CD73, which comprises one or more VHH chains of the nanobody targeting CD73 of the first aspect of the present invention.

In another preferred embodiment, the VHH chain of the nanobody targeting CD73 has an amino acid sequence as shown in SEQ ID NO.4, SEQ ID NO.8, SEQ ID NO.12, SEQ ID NO.16, SEQ ID NO.20, SEQ ID NO.24, SEQ ID NO.28, SEQ ID NO.32, SEQ ID NO.36, SEQ ID NO.46, SEQ ID NO.47, SEQ ID NO.48 or SEQ ID NO.49.

In another preferred embodiment, the antibody is a monomer, a bivalent antibody, and/or a multivalent antibody.

In another preferred embodiment, the antibody is an animal derived antibody, a humanized antibody, a chimeric antibody or a chimeric antigen receptor antibody (CAR).

In another preferred embodiment, the antibody is a humanized antibody, and the VHH chain of the nanobody targeting CD73 has an amino acid sequence as shown in SEQ ID NO.50, SEQ ID NO.51 or SEQ ID NO.52.

In another preferred embodiment, the CDR region of the humanized antibody comprises 1, 2, or 3 amino acid changes.

In another preferred embodiment, the animal is a non-human mammal, preferably a mouse, sheep, rabbit or camel.

In another preferred embodiment, the antibody is a double chain antibody or a single chain antibody.

In another preferred embodiment, the antibody is a monoclonal antibody.

In another preferred embodiment, the antibody is a partially or fully humanized monoclonal antibody.

In another preferred embodiment, the number of added, deleted, modified and/or substituted amino acids does not exceed 40%, preferably 20%, and more preferably 10% of the total number of amino acids in the original amino acid sequence.

In another preferred embodiment, the number of added, deleted, modified and/or substituted amino acids is 1-7, preferably 1-3, and more preferably 1.

In another preferred embodiment, the amino acid sequence that is added, deleted, modified and/or substituted with at least one amino acid is an amino acid sequence having a homology of at least 80%.

In another preferred embodiment, the derivative sequence that is added, deleted, modified and/or substituted with at least one amino acid has the function of inhibiting the enzymatic function of CD73 on cell surface or recombinant CD73 protein.

In another preferred embodiment, the antibody is in the form of a drug conjugate.

In another preferred embodiment, the binding affinity EC₅₀ of the nanobody to the extracellular domain of human CD73 protein (CD73-ECD) detected by ELISA ranges from 2.67ng/mL~13.48ng/mL, and that of the humanized antibody ranges from 2.1ng/mL~2.5ng/mL; or the binding constant KD value measured by ForteBio surface plasmon resonance (SPR) ranges from 0.795~1.997nM, and that of the humanized antibody ranges from 0.472nM~ 1.297nM.

In another preferred embodiment, the antibody has one or more properties selected from the group consisting of:
(a) inhibiting the activity of CD73 in catalyzing the hydrolysis of adenosine monophosphate (AMP) into adenosine;
(b) specifically binding to CD73 on a tumor cell, and/or an immune/stromal cell in the tumor microenvironment;
(c) inhibiting the activity of CD73 in a tumor/a tumor microenvironment in catalyzing AMP hydrolysis;
(d) inhibiting tumor cell migration or metastasis;
(e) inhibiting tumor growth and improving the anti-tumor efficacy of combination drug therapy;
(f) promoting the proliferation, survival and function of an immune cell, thereby improving the effect of tumor immunity;
(g) binding to a tumor cell and being internalized into an intracellular lysosome;
(h) having the properties of penetrating the blood-brain barrier and being distributed in brain;
(i) having excellent therapeutic effect on intracranial tumor models;
(j) having excellent therapeutic effect on various solid tumors.

In the third aspect of the present invention, it provides a multispecific antibody, wherein the multispecific antibody comprises: the nanobody targeting CD73 of the first aspect of the present invention or the antibody targeting CD73 of the second aspect of the present invention.

In another preferred embodiment, the multispecific antibody further comprises a second antigen binding region targeting a target selected from the group consisting of: EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, BCMA, GLP-1, Trop2, TIGIT, LAG-3, FGL1, TLR7, or a combination thereof.

In another preferred embodiment, the second antigen binding region is a nanobody.

In another preferred embodiment, the multispecific antibody comprises one or more second antigen binding regions.

In another preferred embodiment, the multispecific antibody further comprises an antibody Fc segment.

In another preferred embodiment, the antigen binding region is an antibody or an antibody fragment, wherein the antibody fragment comprises: (i) Fab fragment; (ii) F(ab')₂ fragment; (iii) Fd fragment; (iv) Fv fragment; (v) single-chain Fv (scFv) molecule; (vi) dAb fragment.

In the fourth aspect of the present invention, it provides a recombinant protein, wherein the recombinant protein comprises:
(i) the nanobody targeting CD73 of the first aspect of the invention, or the antibody targeting CD73 of the second aspect of the invention; and
(ii) an optional polypeptide molecule or fragment with therapeutic function; and/or
(iii) an optional functional domain for improvement of the physicochemical properties or druggability of the protein.

In another preferred embodiment, the improvement of the physicochemical properties or drugability of the protein includes prolonging the half-life of the nanobody targeting CD73.

In another preferred embodiment, the recombinant protein further comprises: (iv) an optional tag sequence for facilitating expression and/or purification.

In another preferred embodiment, the tag sequence is selected from the group consisting of: 6His tag, GGGS sequence, FLAG tag.

In another preferred embodiment, the recombinant protein is a monomer, dimer, or polymer.

In another preferred embodiment, the polypeptide molecule or fragment withtherapeutic function includes but not limited to: polypeptide molecule or fragment targeting EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, BCMA, GLP-1, Trop2, TIGIT, LAG-3, FGL1, TLR7.

In another preferred embodiment, the polypeptide molecule or fragment with therapeutic function includes but not limited to an insulin, IL-2, interferon, calcitonin, GHRH peptide, intestinal peptide analog, albumin, antibody fragment, and cytokine.

In another preferred embodiment, the recombinant protein (or polypeptide) comprises a fusion protein.

In another preferred embodiment, the fusion protein includes a multispecific antibody, and a chimeric antibody.

In another preferred embodiment, the functional domain for improvement of the physicochemical properties or druggability of a protein includes an Fc segment, an anti-albumin nanobody (HLE), and an albumin binding domain (ABD).

In another preferred embodiment, the fusion protein has the following elements from the N-terminus to C-terminus:

A-B;

wherein the element A is the nanobody targeting CD73; the element B is an Fc segment, an albumin binding domain (ABD) or an anti-albumin nanobody (HLE);
"-" represents a peptide bond or linker.

In another preferred embodiment, the VHH chain of the nanobody targeting CD73 is selected from the group consisting of: the amino acid sequence as shown in SEQ ID NO.4, SEQ ID NO.8, SEQ ID NO.12, SEQ ID NO.16, SEQ ID NO.20, SEQ ID NO.24, SEQ ID NO.28, SEQ ID NO.32, SEQ ID NO.36, SEQ ID NO.46, SEQ ID NO.47, SEQ ID NO.48 or SEQ ID NO.49.

In another preferred embodiment, the linker is (G4S)n, wherein n is a positive integer of 1-4; and the linker may further comprise one or more cysteine residue modifications.

In another preferred embodiment, the Fc segment is a human IgG Fc segment.

In another preferred embodiment, the element B is an Fc segment, and the amino acid sequence of the fusion protein is selected from the group consisting of: the amino acid sequences as shown in SEQ ID NO.37, SEQ ID NO.38, SEQ ID NO.39, SEQ ID NO.40, SEQ ID NO.41, and SEQ ID NO.42.

In another preferred embodiment, the fusion protein is selected from the group consisting of: 3-D7, 5-E11, 4-D04, 4-B02, 1-E7, 2-B10, 2-G9, 3-A3, 3-F1, 3-D7-FC1, 3-D7-FC2, 3-D7-FC3, 5-E11-FC1, 5-E11-FC2, 5-E11-FC3, 3-D7-HLE, 3-D7-ABD, 5-E11-ABD, 5-E11ASCS-FC1, 3D7-HM7-FC1, 3D7-HM8-FC1, 3D7-HM9-FC1, 3D7-HM9-ABD.

In the fifth aspect of the present invention, it provides a CAR construct, wherein the antigen binding region of the CAR construct is the VHH chain of the nanobody of the first aspect of the present invention.

In the sixth aspect of the present invention, it provides a recombinant immune cell, wherein the immune cell expresses the exogenous CAR construct of the fifth aspect of the present invention.

In another preferred embodiment, the immune cell is selected from the group consisting of: a NK cell and a T cell.

In another preferred embodiment, the immune cell is derived from human or non-human mammal (such as mouse).

In the seventh aspect of the present invention, it provides an immunoconjugate, wherein the immunoconjugate comprises:
(a) an antibody moiety, wherein the antibody moiety is the nanobody targeting CD73 of the first aspect of the invention or the antibody targeting CD73 of the second aspect of the invention; and
(b) a coupling moiety coupled to the nanobody moiety, wherein the coupling moiety is selected from the group consisting of: a detectable marker, a drug, a toxin, a cytokine, an enzyme, a protein degrader, an oligonucleotide or a combination thereof.

In another preferred embodiment, the immunoconjugate is a nanobody-drug conjugate.

In another preferred embodiment, the nanobody moiety is coupled to the coupling moiety via a chemical bond or a linker.

In another preferred embodiment, the coupling moiety is a chemical marker and biomarker.

In another preferred embodiment, the chemical marker is an isotope, an immunotoxin and/or a chemical drug.

In another preferred embodiment, the biological marker is a biotin, an avidin or an enzyme marker.

In another preferred embodiment, the coupling moiety is a drug or a toxin.

In another preferred embodiment, the drug is a cytotoxic drug.

In another preferred embodiment, the cytotoxic drug is selected from the group consisting of: an antitubulin drug, DNA sulcus binding reagent, DNA replication inhibitor, alkylation reagent, antibiotic, folic acid antagonist, antimetabolite, chemosensitizer, topoisomerase inhibitor, vinca alkaloids, and a combination thereof.

Particularly useful cytotoxic drug includes, for example, DNA sulcus binding reagent, DNA alkylation reagent, and tubulin inhibitor. The typical cytotoxic drug includes, such as auristatins, camptothecins, duocarmycins, etoposides, maytansines and maytansinoids (such as DM1 and DM4), taxanes, benzodiazepines, or benzodiazepines containing drugs (for example pyrrolo [1,4] benzodiazepine (PBD), indolinobenzodiazepines, and oxazolidinobenzodiazepines, vinca alkaloids, or a combination thereof.

In another preferred embodiment, the toxin is selected from the group consisting of:
auristatin (e.g., auristatin E, auristatin F, MMAE and MMAF), chlortetracycline, maytansinoid, ricin, ricin A-chain, combretastatin, duocarmycin, dolastatin, adriamycin, daunorubicin, paclitaxel, cisplatin, cc1065, ethidium bromide, mitomycin, etoposide, tenoposide, vincristine, vinblastine, colchicine, dihydroxy anthrax dione, actinomycin, diphtheria toxin, pseudomonas exotoxin (PE) A, PE40, abrin, abrin A chain, modeccin A chain, α-octococcus, gelonin, mitogellin, retstrictocin, phenomycin, enomycin, curicin, crotonin, calicheamicin, Sapaonaria officinalis inhibitors, glucocorticoids, or a combination thereof.

In another preferred embodiment, the coupling moiety is a detectable marker.

In another preferred embodiment, the detectable marker comprises a radionuclide, and the radionuclide comprises:
(i) a diagnostic isotope selected from the group consisting of: Tc-99m, Ga-68, F-18, I-123, I-125, 1-131, In-111, Ga-67, Cu-64, Zr-89, C-11, Lu-177, Re-188, or a combination thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133Yb-169, Yb-177, or a combination thereof.

In another preferred embodiment, the protein degrader is a degrader of a tumor-related protein selected from the group consisting of: EGFR, NF-κB, RIPK2, BCR-ABL, HER2, c-Met, TBK1, CDK, ALK, Akt, CK2, ERK1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3 FRS2, and RAS (such as KRAS, HRAS and NRAS).

In another preferred embodiment, the KRAS includes KRAS-G12C, KRAS-G 12D, KRAS-G12V, etc.

In another preferred embodiment, the protein degrader includes PROTAC (proteolysis targeting chimera).

In another preferred embodiment, the PROTAC targets EGFR, KRAS (including KRAS-G 12C, KRAS-G 12D, KRAS-G12V, etc.).

In another preferred embodiment, the oligonucleotide includes an antisense oligonucleotide (ASO), a small interfering RNA (siRNA), a micro RNA (miRNA), a small activating RNA (saRNA), a messenger RNA (mRNA) or a RNA aptamer.

In another preferred embodiment, the coupling moiety is selected from: a fluorescent or luminescent marker, a radioactive marker, MRI (magnetic resonance imaging) or CT (computed tomography) contrast agent, or an enzyme capable of producing detectable product, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), chemotherapeutic agent (e.g., cisplatin) or a nanoparticle in any form, etc.

In another preferred embodiment, the immunoconjugate comprises: a nanobody targeting CD73 of the first aspect of the present invention or an antibody targeting CD73 of the second aspect of the present invention with multivalency.

In another preferred embodiment, the multivalency means that the amino acid sequence of the immunoconjugate comprises multiple repeats of the nanobody targeting CD73 of the first aspect of the present invention or the antibody targeting CD73 of the second aspect of the present invention.

In another preferred embodiment, the detection is *in vivo* detection or *in vitro* detection.

In another preferred embodiment, the immunoconjugate is used for diagnosing and/or treating a tumor that expresses CD73 protein.

In another preferred embodiment, the antibody-drug conjugate ADC is as shown in the following molecular formula: 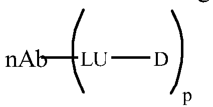 wherein:
nAb is the nanobody targeting CD73.
LU is a linker (also called a connector);
D is a drug;
and the subscript p is a value selected from 1-8.

In another preferred embodiment, LU is a linker selected from maleimidocaproyl (MC), maleimide (MAL), succinimidyl 4-(N-maleimidomethyl) cyclohexane-1-arboxylate) (SMCC) linker linked to the antibody moiety, and comprises one or more of valine-citrulline (VC), valine-alanine (VA), glycine-glycine-phenylalanine-glycine (GGFG), alanine-alanine-alanine (AAA), p-aminobenzyloxycarbonyl (PAB), and polyethylene glycol (PEG).

In another preferred embodiment, the antibody is covalently bound to the linker by reacting with a moiety selected from the group consisting of maleimidyl hexanoyl (MC), maleimide (MAL), succinimidyl 4-(N-maleimidomethyl)cyclohexane-1-carboxylate) (SMCC), etc.

In another preferred embodiment, D is a compound with anti-tumor activity selected from the group consisting of:
(i) a tubulin inhibitor, such as maytansine derivative (DM1, DM4), monomethyl auristatin E (MMAE), monomethyl auristatin F (MMAF);
(ii) a toxin that acts on DNA, such as duocarmycin and pyrrolobenzodiazepine (PBD);
(iii) a topoisomerase inhibitor, camptothecin, SN38, Exatecan, Dxd.

In another preferred embodiment, the compound LU-D is selected from the group consisting of:

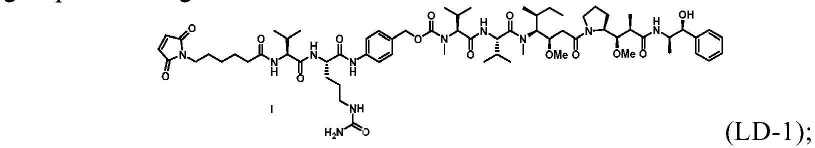

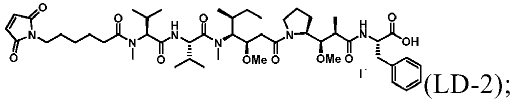

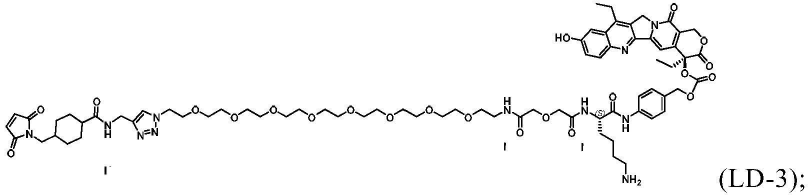

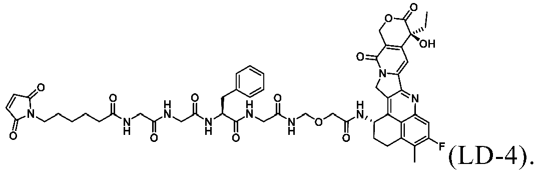

In the eighth aspect of the present invention, it provides a pharmaceutical composition comprising:
(i) the nanobody targeting CD73 of the first aspect of the invention, the antibody targeting CD73 of the second aspect of the invention, the multispecific antibody of the third aspect of the invention, the recombinant protein of the fourth aspect of the invention, the recombinant immune cell of the sixth aspect of the invention, or the immunoconjugate of the seventh aspect of the invention; and
(ii) a pharmaceutically acceptable carrier.

In another preferred embodiment, the pharmaceutical composition includes a single drug, a compound drug, or a synergistic drug.

In another preferred embodiment, the pharmaceutical composition further comprises other biologically active substances, such as drugs for treating tumors.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spraying and aerosol inhalation.

In another preferred embodiment, the dosage form of the pharmaceutical composition is selected from the group consisting of: liquid, solid, or gel.

In another preferred embodiment, the pharmaceutical composition is a liquid formulation.

In another preferred embodiment, the pharmaceutical composition is an injection.

In the ninth aspect of the present invention, it provides a use of an active ingredient, wherein the active ingredient is selected from the group consisting of: the nanobody targeting CD73 of the first aspect of the present invention, the antibody targeting CD73 of the second aspect of the present invention, the multispecific antibody of the third aspect of the present invention, the recombinant protein of the fourth aspect of the present invention, the recombinant immune cell of the sixth aspect of the present invention, the immunoconjugate of the seventh aspect of the present invention, or a combination thereof, wherein the active ingredient is used for (a) preparing a detection reagent, a detection plate or a kit; and/or (b) preparing a medicine for the prevention and/or treatment of a CD73-related disease.

In another preferred embodiment, the detection reagent, detection plate or kit is used in:
(1) detection of CD73 protein in a sample; and/or
(2) detection of endogenous CD73 protein in a tumor cell; and/or
(3) detection of a tumor cell expressing CD73 protein.

In another preferred embodiment, the type of the detection includes but is not limited to flow cytometry, cell immunofluorescence detection, enzyme-linked immunosorbent assay, immunoblotting detection, etc.

In another preferred embodiment, the detection reagent, detection plate or kit is used for diagnosing a CD73-related disease.

In another preferred embodiment, the drug is used for treating or preventing a tumor, tumor migration, or tumor resistance with a high CD73 expression.

In another preferred embodiment, the tumor resistance includes: resistance to a tumor immunotherapy drug, resistance to a tumor targeted therapy drug, resistance to conventional tumor chemotherapy, and insensitivity to radiotherapy.

In another preferred embodiment, the drug is used for a purpose selected from the group consisting of:
(a) inhibiting the activity of CD73 in catalyzing the hydrolysis of adenosine monophosphate (AMP) into adenosine;
(b) specifically binding to CD73 on a tumor cell, and/or an immune/stromal cell in the tumor microenvironment;
(c) inhibiting the activity of CD73 in a tumor/tumor microenvironment in catalyzing AMP hydrolysis;
(d) inhibiting tumor cell migration or metastasis;
(e) inhibiting tumor growth and improving the anti-tumor efficacy of combination drug therapy;
(f) promoting the proliferation, survival and function of an immune cell, thereby improving the effect of tumor immunity.

In another preferred embodiment, the CD73-related disease is selected from the group consisting of: a cancer, autoimmune disease, metabolic-related disease, infectious disease, or a combination thereof.

In another preferred embodiment, the CD73-related disease comprises: tumor occurrence, growth and/or metastasis.

In another preferred embodiment, the cancer comprises a solid tumors and a hematologic cancer.

In another preferred embodiment, the cancer is a tumor with a high expression of CD73.

In another preferred embodiment, the tumor with a high expression of CD73 is selected from the group consisting of: breast cancer, lung cancer, pancreatic cancer, ovarian cancer, prostate cancer, colorectal cancer, glioma, melanoma, leukemia, lymphoma, or a combination thereof.

In another preferred embodiment, the cancer is a drug-resistant tumor.

In another preferred embodiment, the tumor with a high CD73 expression refers to the ratio of the level L1 of CD73 transcript and/or protein in a tumor tissue to the level L0 of transcript and/or protein in a normal tissue, and L1/L0 is ≥ 2, preferably ≥ 3.

In the tenth aspect of the present invention, it provides a polynucleotide encoding a polypeptide selected from group consisting of:
(1) the nanobody targeting CD73 of the first aspect of the invention, the antibody targeting CD73 of the second aspect of the invention, or the multispecific antibody of the third aspect of the invention;
(2) the recombinant protein of the fourth aspect of the present invention; or
(3) the CAR construct of the fifth aspect of the present invention.

In another preferred embodiment, the polynucleotide comprises RNA, DNA or cDNA.

In the eleventh aspect of the present invention, it provides a vector comprising the polynucleotide of the tenth aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In the twelfth aspect of the present invention, it provides a genetically engineered host cell comprising the vector of the eleventh aspect of the present invention or having the polynucleotide of the tenth present aspect of the invention integrated into its genome.

In the thirteenth aspect of the present invention, it provides a method (including diagnostic or non-diagnostic method) for detection of CD73 in a sample *in vitro,* comprising the steps of:
(1) contacting the sample with the nanobody targeting CD73 of the first aspect of the invention, the antibody targeting CD73 of the second aspect of the invention, or the immunoconjugate of the seventh aspect of the invention *in vitro*;
(2) detecting whether an antigen-antibody complex is formed, wherein the formation of the complex indicates the presence of CD73 in the sample.

In another preferred embodiment, the detection includes diagnostic or non-diagnostic detection.

In the fourteenth aspect of the present invention, it provides a detection plate comprising: a substrate (support plate) and a test strip, wherein the test strip contains the nanobody targeting CD73 of the first aspect of the present invention or the antibody targeting CD73 of the second aspect of the present invention or the immunoconjugate of the seventh aspect of the present invention.

In the fifteenth aspect of the present invention, it provides a kit comprising:
(1) a first container containing the nanobody targeting CD73 of the first aspect of the invention or the antibody targeting CD73 of the second aspect of the invention; and/or
(2) a second container containing a secondary antibody against the nanobody targeting CD73 of the first aspect of the present invention or the antibody targeting CD73 of the second aspect of the present invention;
alternatively, the kit contains the detection plate of the fourteenth aspect of the present invention.

In the sixteenth aspect of the present invention, it provides a method for preparing a recombinant polypeptide, comprising:
(a) culturing the host cell of the twelfth aspect of the present invention under a condition suitable for expression;
(b) isolating the recombinant polypeptide from the culture, wherein the recombinant polypeptide is the nanobody targeting CD73 of the first aspect of the invention, the antibody targeting CD73 of the second aspect of the invention, the multispecific antibody of the third aspect of the invention, or the recombinant protein of the fourth aspect of the invention.

In the seventeenth aspect of the present invention, it provides a method for treating a CD73-related disease, comprising: administering to a subject in need the nanobody targeting CD73 of the first aspect of the present invention, the antibody targeting CD73 of the second aspect of the present invention, the multispecific antibody of the third aspect of the present invention, the recombinant protein of the fourth aspect of the present invention, the recombinant immune cell of the sixth aspect of the present invention, the immunoconjugate of the seventh aspect of the present invention, the pharmaceutical composition of the eighth aspect of the present invention, or a combination thereof.

In another preferred embodiment, the method further comprises: administering to a subject in need other drugs or treatment methods for combined treatment.

In another preferred embodiment, the other drugs or treatment methods include: an anti-tumor immunotherapy drug, tumor targeted drug, tumor chemotherapy drug, and tumor radiotherapy.

In another preferred embodiment, the anti-tumor immunotherapy drug comprises PD-1 and PD-L1 monoclonal antibody.

In the eighteenth aspect of the present invention, it provides a method for preparing a chimeric antibody, comprising the steps of:
cloning the nucleotide sequence of the alpaca-derived VHH sequence of the nanobody targeting CD73 of the first aspect of the present invention into an expression vector comprising the nucleotide sequence of the human antibody constant region, and expressing the human-alpaca chimeric antibody by transfecting animal cells.

In the nineteenth aspect of the present invention, it provides a method for preparing a humanized antibody, comprising the steps of:
the VHH chain of the nanobody targeting CD73 of the first aspect of the present invention is implanted into a nucleotide sequence template comprising the FR region of a human antibody, which is then cloned into an expression vector comprising a human antibody constant region, and the humanized antibody is expressed by transfecting animal cells.

In the twentieth aspect of the present invention, it provides a method for inhibiting tumor cell growth and migration, comprising the steps of administering to a subject in need the nanobody targeting CD73 of the first aspect of the present invention, the antibody targeting CD73 of the second aspect of the present invention, or the nanobody-drug conjugate of the present invention, or a CAR-T cell of the nanobody, or a combination thereof.

In the twenty-first aspect of the present invention, it provides a method for inhibiting tumor growth in a model animal, comprising the steps of administering to a subject in need the nanobody targeting CD73 of the first aspect of the present invention, the antibody targeting CD73 of the second aspect of the present invention, the nanobody-drug conjugate of the present invention, or a CAR-T cell of the nanobody.

In another preferred embodiment, the drug can be administered alone, or in combination with tumor immunotherapy, a tumor-targeted drug, a cytotoxic drug, and radiotherapy.

In the twenty-second aspect of the present invention, it provides a preparation method of the nanobody-drug conjugate of the seventh aspect of the present invention, comprising the steps of:
(1) reacting the antibody with a reducing agent in a buffer to obtain a reduced antibody;
(2) cross-linking (coupling) the reduced antibody obtained in step (1) with the linker-drug conjugate of formula DL in a mixture of buffer and organic solvent to obtain antibody-drug conjugates 1a, 1b, 1c or 1d with different DAR values.

In another preferred embodiment, the cross-linking reaction of the preparation method is as shown in Figure 46.

In another preferred embodiment, the antibody in step (1) is reduced with a reducing agent, so that the interchain disulfide bonds of the antibody are reduced to produce thiol groups.

In another preferred embodiment, the reducing agent in step (1) is tris(2-carboxyethyl)phosphine hydrochloride (TCEP), beta-mercaptoethanol, beta-mercaptoethylamine hydrochloride, or dithiothreitol (DTT).

In another preferred embodiment, the buffer is selected from the group consisting of: potassium dihydrogen phosphate-sodium hydroxide (KH₂PO₄-NaOH)/sodium chloride (NaCl)/diethyltriaminepentaacetic acid (DTPA) buffer, disodium hydrogen phosphate-citric acid/sodium chloride (NaCl)/diethyltriaminepentaacetic acid (DTPA), boric acid-borax/sodium chloride (NaCl)/diethyltriaminepentaacetic acid (DTPA), histidine-sodium hydroxide/sodium chloride (NaCl)/diethyltriaminepentaacetic acid (DTPA), and PBS/diethyltriaminepentaacetic acid (DTPA).

In another preferred embodiment, in the step (2), the organic solvent in the reaction solution is no more than 15% by volume.

In another preferred embodiment, the organic solvent in step (2) is selected from the group consisting of: acetonitrile (ACN), dimethylformamide (DMF), dimethylacetamide (DMA), and dimethyl sulfoxide (DMSO).

Then the linker drug (pre-dissolved in acetonitrile (ACN), dimethyl sulfoxide (DMSO), dimethylformamide (DMF) or diethylacetamide (DMA) at 10 mg/ml) is added. It should be ensured that the organic solvent in the reaction solution is no more than 15% by volume. The coupling reaction is under 0-37 °C and being stirred for 2-4 hours. If TCEP is used for reduction, it is also possible to directly add the substituted maleimide compound for coupling without removing the remaining TCEP.

The mixture of the coupling reaction is purified by filtration with sodium succinate/NaCl buffer or histidine-acetic acid/sucrose gel using a desalting column, and the peak samples are collected according to the UV280 ultraviolet absorption value. Or ultrafiltration is performed several times. Then, the product is filtered and sterilized, and stored at low temperature.

The drug-antibody conjugates obtained have a relatively uniform drug antibody conjugate ratio (DAR). For NDCs with certain differences in DAR, if a sample with better uniformity is required, further separation and purification can be performed using, but not limited to, the following methods: hydrophobic interaction chromatography (HIC), size exclusion chromatography (SEC), and ion exchange chromatography (IEC).

It should be understood that within the scope of the present invention, each technical feature of the present invention described above and in the following (such as examples) may be combined with each other to form a new or preferred technical solution, which is not listed here due to space limitations.

### DESCRIPTION OF THE DRAWINGS

Figure 1 shows the binding affinity constants (KD) of four chimeric nanobodies 3-D7, 5-E11, 4-D04, and 4-B02 to human CD73-ECD determined by surface plasmon resonance (SPR) analysis.
Figure 2 shows the ELISA binding affinity (EC₅₀) of 3-D7, 5-E11, 4-D04, and 4-B02 to CD73-ECD.
Figure 3 shows the inhibitory activity (IC₅₀) of 3-D7, 5-E11, 4-D04, and 4-B02 on the catalytic function of the recombinant human CD73 enzyme.
Figure 4 shows the binding affinity (EC₅₀) test results of 3-D7, 5-E11, 4-D04, and 4-B02 to CD73 on the surface of MDA-MB-231 (Figure 4A) or NCI-H1299 (Figure 4B) cells.
Figure 5 shows the binding affinity (EC₅₀) test results of 3-D7, 5-E11, 4-D04, and 4-B02 to CD73 on the surface of PC9 (Figure 5A) and NCI-H1975 (Figure 5B) cells.
Figure 6 shows the binding affinity (EC₅₀) test results of 3-D7, 4-D04, 5-E11, and 4-B02 to CD73 on the surface of SW1573 (Figure 6A) and NCI-H1373 (Figure 6B) cells.
Figure 7 shows the binding affinity (EC₅₀) test results of 3-D7, 5-E11, 4-D04, and 4-B02 to CD73 on the surface of HCC-44 cells.
Figure 8 shows the inhibitory activity of 3-D7, 5-E11, 4-D04, and 4-B02 on the catalytic function of CD73 enzyme on the surface of MDA-MB-231 cells, and the inhibition curves and IC₅₀ values are demonstrated.
Figure 9 shows that 3-D7, 5-E11, 4-D04, and 4-B02 can effectively reverse the inhibitory effect of adenosine monophosphate (AMP) on the proliferation of human T lymphocytes. In the experiment, human CD3+ T cells were obtained by sorting, and cultured for 5 days, and then the cell proliferation rate and EC₅₀ value were calculated.
Figure 10 shows that 3-D7, 5-E11, 4-D04, and 4-B02 can effectively reverse the inhibitory effect of AMP on the expression of INF-g in human T lymphocytes. In the experiment, human CD3+ T cells were obtained by sorting, and cultured for 5 days, and then the T cell culture supernatant was detected.
Figure 11 shows the ELISA binding activity of chimeric nanobodies 1-B11, 1-E7, 2-B10, 2-G9, 3-A3, and 3-F1 to human CD73-ECD. 100 µL, 10 µL, and 1 µL of the supernatant of HEK293F cells transiently transfected and cultured for 2 days were used for detection.
Figure 12 shows the binding activity of chimeric nanobodies 1-B11, 1-E7, 2-B10, 2-G9, 3-A3, and 3-F1 to CD73 on the surface of MDA-MB-231 cells. 100 µL, 10 µL, and 1 µL of the supernatant of HEK293F cells transiently transfected and cultured for 2 days were used for detection.
Figure 13 shows the binding of chimeric nanobodies 3-D7, 5-E11, 4-D04, 4-B02 to MDA-MB-231 cells, and resulting in their internalization into intracellular lysosomes. The results were observed under a fluorescence microscope after the antibodies (5 µg/mL) were incubated with cells at 4°C for 1 hour, or at 37°C for 4 hours.
Figure 14 shows the detection results of the proliferation inhibitory activity of chimeric nanobody-drug conjugates (NDC) 3-D7-MMAE, 5-E11-MMAE and the control Hu001-MMAE (CD73-ADC) to breast cancer cells MDA-MB-453 (Figure 14A) and MDA-MB-231 (Figure 14B).
Figure 15 shows the detection results of the proliferation inhibitory activity of 3-D7-MMAE, 5-E11-MMAE and the control Hu001-MMAE to lung cancer cells PC9 (Figure 15A) and NCI-H1975 (Figure 15B).
Figure 16 shows the detection results of the proliferation inhibitory activity of 3-D7-MMAE, 5-E11-MMAE and the control Hu001-MMAE to lung cancer cells NCI-H441 (Figure 16A) and SW1573 (Figure 16B).
Figure 17A shows the number of live cells in a fixed volume after human T lymphocytes being incubated with different concentrations of 3-D7-MMAE and 5-E11-MMAE for 5 days, and the inhibition curve was drawn based on the proliferation rate (relative to the vehicle/buffer); Figure 17B shows that 3-D7-MMAE and 5-E11-MMAE can effectively reverse the proliferation inhibition of adenosine monophosphate (AMP) to human T lymphocytes. In the experiment, CD3+ human T lymphocytes were obtained by sorting, and cultured for 5 days, and then the cell proliferation rate was calculated.
Figure 18 shows that 3-D7-MMAE and 5-E11-MMAE can effectively reverse the inhibitory effect of AMP on the expression of INF-g in human T lymphocytes. In the experiment, CD3⁺ human T cells were obtained by sorting, and cultured for 5 days, and then the T cell culture supernatant was detected.
Figure 19A shows the SDS-PAGE of the chimeric nanobodies 3-D7 after expression and purification, corresponding to SEQ ID NO. 37, SEQ ID NO.38, SEQ ID NO.39; Figure 19B is the SDS-PAGE of the 3-D7 recombinant fusion proteins after purification, corresponding to SEQ ID NO.43, SEQ ID NO.44.
Figure 20A shows the SDS-PAGE of the chimeric nanobodies 5-E11 after expression and purification, corresponding to SEQ ID NO.40, SEQ ID NO.41, SEQ ID NO.42; Figure 20B is the SDS-PAGE of the 5-E11 recombinant fusion protein after purification, corresponding to SEQ ID NO.45.
Figure 21 shows the mass spectrum of the chimeric nanobody 3-D7-vc-MMAE.
Figure 22 shows the mass spectrum of the chimeric nanobody 5-E11-vc-MMAE.
Figure 23 shows the mass spectrum of the chimeric nanobody 4-D04-vc-MMAE.
Figure 24 shows the ELISA binding activity of 5-E11 and three mutants thereof (5-E11AS, 5-E11GS, 5-E11QS) to CD73-ECD at gradient concentrations.
Figure 25 shows the ELISA binding activity of 5-E11 and mutant 5-E11ASCS to human CD73-ECD at gradient concentrations, and the binding affinity (EC₅₀) was demonstrated.
Figure 26 shows the binding affinity (EC₅₀) of 5-E11 and mutant 5-E11ASCS to CD73 on the surface of SW1573 cells at gradient concentrations.
Figure 27 shows the binding affinity constants (KD) of 3-D7 and its three humanized antibodies 3D7-HM7-FC1, 3D7-HM8-FC1, and 3D7-HM9-FC1 to human CD73-ECD determined by surface plasmon resonance (SPR) analysis.
Figure 28 shows the ELISA binding activity of humanized antibodies 3D7-HM9-FC1 and 3D7-HM9-FCWT to human CD73-ECD at gradient concentrations, and the binding affinity (EC₅₀) was demonstrated.
Figure 29 shows the ELISA binding activity of humanized antibodies 3D7-HM9-FC1 and 3D7-HM9-FCWT to cynomolgus monkey CD73-ECD at gradient concentrations, and the binding affinity (EC₅₀) was demonstrated.
Figure 30 shows the inhibitory activity (EC₅₀) of 3-D7 and humanized antibodies 3D7-HM7-FC1, 3D7-HM8-FC1, and 3D7-HM9-FC1 to human recombinant CD73 enzyme at gradient concentrations.
Figure 31 shows the binding affinity (EC₅₀) of 3-D7 and humanized antibodies 3D7-HM7-FC1, 3D7-HM8-FC1, and 3D7-HM9-FC1 to CD73 on the surface of H299 cells at gradient concentrations as determined by FACS.
Figure 32 shows the inhibitory activity of humanized antibodies 3D7-HM9-FC1, 3D7-HM9-FCWT, and 3D7-HM9-ABD on the catalytic function of CD73 enzyme on the surface of MDA-MB-231 cells, and the inhibition curve and IC₅₀ values were demonstrated.
Figure 33 shows that humanized antibodies 3D7-HM9-FC1 and 3D7-HM9-ABD can effectively reverse the inhibitory effect of adenosine monophosphate (AMP) on the proliferation of human T lymphocytes. In the experiment, human CD3+ T cells were obtained by sorting, and cultured for 5 days, and then the cell proliferation rate and EC₅₀ value were calculated.
Figure 34 shows the number of live cells at a fixed volume after human T lymphocytes incubated with different concentrations of 3D7-HM9-FC1-MMAE and 3D7-HM9-ABD-MMAE for 5 days. The inhibition curve was plotted based on the proliferation rate (relative to the vehicle/buffer).
Figure 35 shows that 3D7-HM9-FC1-MMAE can effectively reverse the inhibitory effect of adenosine monophosphate (AMP) on the proliferation of human T lymphocytes. In the experiment, CD3+ human T lymphocytes were obtained by sorting, and cultured for 5 days and then the cell proliferation rate was calculated.
Figure 36 shows a hydrophobic interaction chromatography (HIC) of 3D7-HM9-FC1 coupled to VC-MMAE conjugate NDC.
Figure 37 shows a hydrophobic interaction chromatography (HIC) of conjugate NDC 3D7-HM9-ABD coupled with VC-MMAE.
Figure 38 shows the killing effect of intermittent administration of 3D7-FC1-MMAE, 5E11-FC1-MMAE, and Hu001-MMAE on H1975 and SW1573 cells.
Figure 39 shows the efficacy of a single dose (3 mg/kg intravenous injection) of 3D7-FC1-MMAE and 5E11-FC1-MMAE in a PC9 lung cancer subcutaneous transplant tumor model.
Figure 40 shows the efficacy of 3D7-FC1-MMAE, 5E11-FC1-MMAE (single administration in day 13), and 3D7-ABD-MMAE (twice administration in day 13 and day 20) injected intravenously at 3 mg/kg in a HCC44 lung cancer orthotopic tumor model of nude mouse.
Figure 41 shows the efficacy of a single intravenous injection of 3 mg/kg of 3D7-HM9-FCWT-MMAE (day 20) in a HCC44 lung cancer orthotopic tumor model of nude mouse.
Figure 42 shows a detection of the intracranial absorption distribution of antibodies marked with isotope I¹²⁵ after intravenous injection in mice.
Figure 43 shows the efficacy of a single intravenous injection of 5 mg/kg of 3D7-FC1-MMAE (in day 12) in a H1975 lung cancer intracranial model of nude mice.
Figure 44 shows the efficacy of a single intravenous injection of 5 mg/kg of 3D7-HM9-FCWT-MMAE (in day 14) in a H1975 lung cancer intracranial model of nude mice.
Figure 45 shows the structural design of the nanobody fusion protein and drug conjugate thereof of the present invention.
Figure 46 shows a cross-linking reaction formula for the preparation method of the nanobody-drug conjugate of the present invention, wherein the antibody-drug conjugates with different DAR values are shown in 1a, 1b, 1c or 1d, respectively.

### Detailed Description

Through extensive and intensive research, the inventors immunized alpaca and established libraries, screened phage display libraries and yeast display libraries in large quantities, and obtained multiple nanobodies such as 3-D7, 5-E11, 4-D04, and 4-B02. The antibodies can bind to human CD73 protein with high specificity, and the EC₅₀ determined by ELISA are 13.48 ng/mL, 8.72 ng/mL, 2.67 ng/mL, and 3.03 ng/mL, respectively. The antibodies have high inhibitory activity against recombinantly expressed or cell-derived human CD73 enzymes, and can effectively reverse the inhibitory effect of AMP on the proliferation of human T lymphocytes. In addition, the nanobody-drug conjugates (CD73-NDCs) designed based on 3-D7, 5-E11, and 4-D04 not only maintain the consistent effects of the antibodies as above-mentioned, but can also specifically kill a tumor with a high expression of CD73, and has shown good tumor inhibition activity and potential to regulate the immune microenvironment in many aspects. The present invention was completed on this basis.

### Terms

As used herein, the terms "nanobody of the invention", "anti-CD73 nanobody of the invention", and "CD73 nanobody of the invention" are used interchangeably and refer to nanobodies that specifically recognize and bind to CD73 (including human CD73). Particularly preferred are nanobodies with VHH chains having amino acid sequences shown in SEQ ID NO. 4, 8, 12, 16, 20, 24, 28, 32, 36, 46, 47, 48 or 49.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the terms "single domain antibody (VHH) " and "nanobody" have the same meaning and can be used interchangeably to refer to cloning the variable region of the heavy chain of the antibody, constructing a nanobody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

As used herein, the term "variable" means that certain portion of the variable region in an antibody differ in sequence, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, involving in the antibody-dependent cytotoxicity of an antibody.

As known to those skilled in the art, immunoconjugates and fusion expression products include: conjugates formed by binding drugs, toxins, cytokines, radionuclides, enzymes, and other diagnostic or therapeutic molecules to the nanobody or a fragment thereof of the present invention. The present invention also includes a cell surface marker or antigen that binds to the anti-CD73 protein antibody or a fragment thereof.

As used herein, the term "heavy chain variable region" is used interchangeably with "V_{H}".

As used herein, the term "variable region" and "complementarity determining region (CDR)" are used interchangeably.

In a preferred embodiment of the present invention, the heavy chain variable region of the antibody includes three complementary determining regions CDR1, CDR2, and CDR3.

In a preferred embodiment of the present invention, the heavy chain of the antibody includes the above-mentioned heavy chain variable region and heavy chain constant region.

In the present invention, the terms "antibody of the present invention", "protein of the present invention", or "polypeptide of the present invention" are used interchangeably, and all refer to a polypeptide that specifically binds to CD73 protein, such as a protein or polypeptide having heavy chain variable regions. They may or may not contain a starting methionine.

The present invention further provides other proteins or fusion expression products having the antibody of the present invention. Specifically, the present invention includes any protein or protein conjugate and fusion expression product (i.e., immunoconjugate and fusion expression product) having a heavy chain containing a variable region, as long as the variable region is identical to or at least 90% homologous, preferably at least 95% homologous to the heavy chain variable region of the antibody of the present invention.

In general, the antigen-binding properties of a nanobody can be described by three specific regions located in the variable region of the heavy chain, referred as variable regions (CDRs), and the segment is divided into four frame regions (FRs). The amino acid sequences of four FRs are relatively conservative and do not directly participate in binding reactions. These CDRs form a loop structure in which the β-sheets formed by the FRs in between are spatially close to each other, and the CDRs on the heavy chain and the CDRs on the corresponding light chain constitute the antigen-binding site of the nanobody. The amino acid sequences of the same type of nanobodies can be compared to determine which amino acids constitute the FR or CDR regions.

The variable regions of the heavy chains of the nanobody of the invention become a particular interest because at least part of them is involved in binding antigens. Thus, the present invention includes those molecules having a nanobody heavy chain variable region with a CDR, provided that their CDRs are 90% or more (preferably 95% or more, the most preferably 98% or more) identical to the CDRs identified herein.

### Anti-CD73 Nanobody

The present invention provides a variety of nanobodies targeting CD73 with high specificity and high affinity, which only comprise a heavy chain, and the heavy chain comprises heavy chain variable region (VH) amino acid sequences (listed in Table-1 and Table-2).

Preferably, the heavy chain variable region (VH) amino acid sequence has CDR1, CDR2, CDR3 of the following polypeptide sequences:
a1) CDR1 is SEQ ID NO.1: GFLLDYYV, SEQ ID NO.5: GFTLDYYN, SEQ ID NO.9: GFTLDYYN, SEQ ID NO.13: GFPLDYYS, SEQ ID NO.17: GFTLDYYN, SEQ ID NO.21: GFTLDDNA, SEQ ID NO.25: GFLLDYYT, SEQ ID NO.29: GFPLDYYT, SEQ ID NO.33: GFTLDYYT;
a2) CDR2 is SEQ ID NO.2:ISGSDRST, SEQ ID NO.6: SSNSGGST (or SEQ ID NO.54: SSASGGST, SEQ ID NO.55: SSGSGGST, or SEQ ID NO.56: SSQSGGST), SEQ ID NO.10: ISSSEGST, SEQ ID NO.14: IGSSDGST, SEQ ID NO.18: ISNSDGST, SEQ ID NO.22: MRTSDGDT, SEQ ID NO.26: ISSSDSSP, SEQ ID NO.30: ISSSDSSP, SEQ ID NO.34; IRSSDSSP;
a3) CDR3 is SEQ ID NO.3: AADPSPVTVQAMCLPRFPVPY, SEQ ID NO.7: AAYRGWHPSLDARVYDY, SEQ ID NO.11: AADRYYYCSLHVSQYDY, SEQ ID NO.15: AALKYYYCSGHEAIYDY, SEQ ID NO.19: AAYRGWHPSLDARVYDY, SEQ ID NO.23: AAALGTYYSGFYYLAGDGMDY, SEQ ID NO.27: ALRANVYDY, SEQ ID NO.31: SLRANVYDY, SEQ ID NO.35: SLTANVYDY;
a4) a sequence formed by adding, deleting, modifying and/or replacing at least one amino acid in any of the above amino acid sequences and having CD73 binding affinity.

In another preferred embodiment, the sequence formed by adding, deleting, modifying and/or replacing at least one amino acid sequence is preferably an amino acid sequence with a homology of at least 80%, preferably at least 85%, more preferably at least 90%, and most preferably at least 95%.

Preferably, the antibody has the function of inhibiting the enzymatic activity of cell surface and recombinant CD73 protein, and the antibody can be rapidly internalized by the cell into the lysosome.

The antibody of the present invention may be a double-chain or single-chain antibody, and may be selected from an animal-derived antibody, a chimeric antibody, a human-animal chimeric antibody, preferably a humanized antibody, and more preferably a fully humanized antibody.

The antibody derivative of the present invention may be a single-chain antibody and/or an antibody fragment, such as Fab, Fab', (Fab')2 or other antibody derivatives known in the art, as well as any one or more of IgA, IgD, IgE, IgG and IgM antibodies or other subtypes of antibodies.

Wherein, the animal is preferably a mammal, such as mouse.

The antibody of the present invention may be a chimeric antibody, humanized antibody, CDR-grafted and/or modified antibody targeting human CD73.

In a preferred embodiment of the present invention, any one or several sequences of the above-mentioned SEQ ID NOs. 1 to 3, SEQ ID NOs. 5 to 7, SEQ ID NOs. 9 to 11, NOs. 13 to 15, NOs. 17 to 19, NOs. 21 to 23, NOs. 25 to 27, NOs. 29 to 31, NOs. 33 to 35, and NOs. 54 to 56, or sequences thereof with CD73 binding affinity after addition, deletion, modification and/or substitution of at least one amino acid, are located in the CDR region of the variable region (VH).

In the above content of the present invention, the number of added, deleted, modified and/or substituted amino acids is preferably no more than 40%, more preferably no more than 35%, more preferably 1-33%, more preferably 5-30%, more preferably 10-25%, more preferably 15-20% of the total number of amino acids in the initial amino acid sequence.

In the above content of the present invention, more preferably, the number of the added, deleted, modified and/or substituted amino acids may be 1-7, more preferably 1-5, more preferably 1-3, more preferably 1-2.

In another preferred embodiment, the original antibody is human-alpaca chimeric antibodies 3-D7, 5-E11, 4-D04, 4-B02, 1-E7, 2-B10, 2-G9, 3-A3, 3-F1.

In another preferred embodiment, the amino acid sequence numbers of the heavy chain variable region (VH) of the chimeric antibody are listed in Table 1.

In another preferred embodiment, the amino acid sequence numbers of the complementary determining region (CDR) of the antibody are listed in Table 2.

The antibody of the present invention may be used in combination for the construction of a CAR construct, a recombinant immune cell comprising a CAR construct, an antibody-drug conjugate, etc., and may further be used for (a) preparing a detection reagent, a detection plate or kit; and/or (b) preparing a medicament for the prevention and/or treatment of a CD73-related disease.

**Table-1: Representative meanings of each sequence in the sequence list of the present invention**

| Sequence No. | Sequence Name | Sequence No. | Sequence Name |
|---|---|---|---|
| SEQ ID NO.1 | 3-D7 CDR1 | SEQ ID NO.29 | 3-A3 CDR1 |
| SEQ ID NO.2 | 3-D7 CDR2 | SEQ ID NO.30 | 3-A3 CDR2 |
| SEQ ID NO.3 | 3-D7 CDR3 | SEQ ID NO.31 | 3-A3 CDR3 |
| SEQ ID NO.4 | 3-D7 VH | SEQ ID NO.32 | 3-A3 VH |
| SEQ ID NO.5 | 5-E11 CDR1 | SEQ ID NO.33 | 3-F1 CDR1 |
| SEQ ID NO.6 | 5-E11 CDR2 | SEQ ID NO.34 | 3-F1 CDR2 |
| SEQ ID NO.7 | 5-E11 CDR3 | SEQ ID NO.35 | 3-F1 CDR3 |
| SEQ ID NO.8 | 5-E11 VH | SEQ ID NO.36 | 3-F1 VH |
| SEQ ID NO.9 | 4-D04 CDR1 | SEQ ID NO.37 | 3-D7-FC1 |
| SEQ ID NO.10 | 4-D04 CDR2 | SEQ ID NO.38 | 3-D7-FC2 |
| SEQ ID NO.11 | 4-D04 CDR3 | SEQ ID NO.39 | 3-D7-FC3 |
| SEQ ID NO.12 | 4-D04 VH | SEQ ID NO.40 | 5-E11-FC1 |
| SEQ ID NO.13 | 4-B02 CDR1 | SEQ ID NO.41 | 5-E11-FC2 |
| SEQ ID NO.14 | 4-B02 CDR2 | SEQ ID NO.42 | 5-E11-FC3 |
| SEQ ID NO.15 | 4-B02 CDR3 | SEQ ID NO.43 | 3-D7-HLE |
| SEQ ID NO.16 | 4-B02 VH | SEQ ID NO.44 | 3-D7-ABD |
| SEQ ID NO.17 | 1-E7 CDR1 | SEQ ID NO.45 | 5-E11-ABD |
| SEQ ID NO.18 | 1-E7 CDR2 | SEQ ID NO.46 | 5-E11 AS VH |
| SEQ ID NO.19 | 1-E7 CDR3 | SEQ ID NO.47 | 5-E11 GS VH |
| SEQ ID NO.20 | 1-E7 VH | SEQ ID NO.48 | 5-E11 QS VH |
| SEQ ID NO.21 | 2-B10 CDR1 | SEQ ID NO.49 | 5-E11 ASCS VH |
| SEQ ID NO.22 | 2-B10 CDR2 | SEQ ID NO.50 | 3D7-HM7 VH |
| SEQ ID NO.23 | 2-B10 CDR3 | SEQ ID NO.51 | 3D7-HM8 VH |
| SEQ ID NO.24 | 2-B10 VH | SEQ ID NO.52 | 3D7-HM9 VH |
| SEQ ID NO.25 | 2-G9 CDR1 | SEQ ID NO.53 | 3D7-HM9-ABD |
| SEQ ID NO.26 | 2-G9 CDR2 | SEQ ID NO.54 | 5-E11 AS CDR2 or 5-E11 ASCS CDR2 |
| SEQ ID NO.27 | 2-G9 CDR3 | SEQ ID NO.55 | 5-E11 GS CDR2 |
| SEQ ID NO.28 | 2-G9 VH | SEQ ID NO.56 | 5-E11 QS CDR2 |

**Table-2: CDR amino acid sequences of nanobodies of the present invention**

| Nanobodies | CDR1 | CDR2 | CDR3 |
|---|---|---|---|
| 3-D7 | GFLLDYYV | ISGSDRST | AADPSPVTVQAMCLPRFPVP Y |
| | SEQ ID NO: 1 | SEQ ID NO: 2 | SEQ ID NO: 3 |
| 5-E11 | GFTLDYYN | SSNSGGST | AAYRGWHPSLDARVYDY |
| | SEQ ID NO: 5 | SEQ ID NO: 6 | SEQ ID NO: 7 |
| 4-D04 | GFTLDYYN | ISSSEGST | AADRYYYCSLHVSQYDY |
| | SEQ ID NO: 9 | SEQ ID NO: 10 | SEQ ID NO: 11 |
| 4-B02 | GFPLDYYS | IGSSDGST | AALKYYYCSGHEAIYDY |
| | SEQ ID NO: 13 | SEQ ID NO: 14 | SEQ ID NO: 15 |
| 1-E7 | GFTLDYYN | ISNSDGST | AAYRGWHPSLDARVYDY |
| | SEQ ID NO: 17 | SEQ ID NO: 18 | SEQ ID NO: 19 |
| 2-B10 | GFTLDDNA | MRTSDGDT | AAALGTYYSGFYYLAGDGMD Y |
| | SEQ ID NO: 21 | SEQ ID NO: 22 | SEQ ID NO: 23 |
| 2-G9 | GFLLDYYT | ISSSDSSP | ALRANVYDY |
| | SEQ ID NO: 25 | SEQ ID NO: 26 | SEQ ID NO: 27 |
| 3-A3 | GFPLDYYT | ISSSDSSP | SLRANVYDY |
| | SEQ ID NO: 29 | SEQ ID NO: 30 | SEQ ID NO: 31 |
| 3-F1 | GFTLDYYT | IRSSDSSP | SLTANVYDY |
| | SEQ ID NO: 33 | SEQ ID NO: 34 | SEQ ID NO: 35 |
| 5-E11AS or | GFTLDYYN | SSASGGST | AAYRGWHPSLDARVYDY |
| 5-E11 ASCS | SEQ ID NO: 5 | SEQ ID NO: 54 | SEQ ID NO: 7 |
| 5-E11GS | GFTLDYYN | SSGSGGST | AAYRGWHPSLDARVYDY |
| | SEQ ID NO: 5 | SEQ ID NO: 55 | SEQ ID NO: 7 |
| 5-E11QS | GFTLDYYN | SSQSGGST | AAYRGWHPSLDARVYDY |
| | SEQ ID NO: 5 | SEQ ID NO: 56 | SEQ ID NO: 7 |

### Recombinant protein (or fusion protein)

In the present invention, it further comprises a recombinant protein (or fusion protein) comprising the CD73 nanobody of the present invention. A preferred fusion protein is a multispecific antibody, which further comprises a second antigen binding region targeting a target selected from the group consisting of: EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, BCMA, GLP-1, Trop2, TIGIT, LAG-3, FGL1, TLR7, or a combination thereof.

Preferably, the multispecific antibody comprises one or more second antigen binding regions, or further comprises a third antigen binding region.

In addition, the recombinant protein (or fusion protein) of the present invention, in addition to the CD73 nanobody of the present invention, further comprises an optional tag sequence that assists in expression and/or purification (e.g., 6His tag, GGGS sequence, FLAG tag); or comprises an optional polypeptide molecule or fragment with therapeutic function; or an optional protein functional domain that assists in physicochemical or pharmaceutical properties (e.g., molecules that may prolong the *in vivo* half-life of a nanobody, such as HLE, ABD).

The present invention includes not only intact antibodies but also fragment(s) of immunologically active antibody or fusion protein(s) formed from antibodies with other sequences. Therefore, the present invention also includes fragments, derivatives and analogs of the nanobodies.

As used herein, the terms "fragment", "derivative", and "analog" refer to a polypeptide that substantially retains the same biological function or activity of a antibody of the invention. Polypeptide fragments, derivatives or analogs of the invention may be (i) polypeptides having one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having CD73 protein binding activity and comprising the CDR regions as discribed above. The term also comprises variant forms of polypeptides having the same function as the antibody of the present invention and comprising the CDR regions as discribed above. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C-terminal and/or N-terminal. For example, in the art, substitutions with amino acids of similar properties generally do not alter the function of the protein. For another example, addition of one or more amino acids to the C-terminal and/or N-terminal usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as a fusion protein containing a nanobody or a fragment thereof. In addition to the almost full-length polypeptide, the present invention also includes a fragment of the nanobody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by replacing at most 10, preferably at most 8, more preferably at most 5, and most preferably at most 3 amino acids with amino acids of similar properties as compared with the amino acid sequence of the antibody of the present invention. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn(N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridizes to the sequence as described above and has at least 50%, preferably at least 70%, and more preferably at least 80% identity between the two sequences. In particular, the present invention relates to a polynucleotide that is hybridizable to the polynucleotide of the present invention under strict conditions. In the present invention, "strict condition" refers: (1) hybridization and elution at lower ionic strength and higher temperature, such as 0.2 × SSC,0.1% SDS, 60 °C; or (2) hybridiztion with denaturing agent, such as 50%(v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence may be obtained by first synthesizing multiple small fragments followed by ligation. In addition, the coding sequence of the heavy chain and the expression tag (such as 6His) may be fused together to form a fusion protein.

### Antibody preparation

The sequence of the DNA molecule for the antibody or a fragment thereof of the present invention may be obtained by conventional techniques, for example, methods such as PCR amplification or genomic library screening. In addition, the sequences encoding light chain and heavy chain may be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence may be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors may be used to transform suitable host cells to enable them to express protein.

The host cell may be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of a monoclonal antibody may be determined by immunoprecipitation or an *in vitro* binding assay (such as radioimmunoassay (RIA) or enzyme-linked immunosorbent assay (ELISA)). The binding affinity of a monoclonal antibody may be determined by, for example, the Scatchard analysis (Munson et al., Anal. Biochem. 107: 220 (1980)).

The antibody according to the present invention may be expressed in a cell or on the cell membrane, or be secreted extracellularly. If necessary, the recombinant protein may be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, super centrifugation, molecular sieve chromatography (gel chromatography), adsorption chromatography, ion exchange chromatography, high performance liquid chromatography (HPLC), and any other liquid chromatography, and a combination thereof.

### Nanobody-drug conjugate (NDC)

The present invention also provides an immunoconjugate based on an antibody of the present invention.

Typically, the antibody-drug conjugate comprises the antibody and an effector molecule, wherein the antibody is conjugated to the effector molecule, and chemical conjugation is preferred. Wherein, the effector molecule is preferably a therapeutically active drug. In addition, the effector molecule may be one or more of a toxic protein, a chemotherapeutic drug, a small-molecule drug or a radionuclide.

The antibody according to the present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that links an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody may be conjugated to a functional agent to form a conjugate of the antibody-functional agent. A functional agent (e.g. a drug, a detection reagent, a stabilizer) is conjugated (covalently linked) to an antibody. A functional agent may be linked to an antibody either directly or indirectly via a linker.

Nanobodies may be conjugated to drugs to form antibody-drug conjugates (NDCs). Typically, a NDC comprises a linker between a drug and an antibody. The linker may be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that may be degraded by protease (e.g. lysosomal protease or endosomal protease) in a cell, or sugar linkers, for example, glucuronide-containing linkers that may be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed when the pH is less than 5.5, such as hydrazone linkers) and linkers that are degraded under a reducing condition (e.g. disulfide-bond linkers). A non-degradable linker typically releases a drug under a condition that the antibody is hydrolyzed by a protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is acetate ion, chloride or nitrate ion; and polymethylene dimethyl sulfide thiosulfonate. The linker may include, for example, a maleimide linked to an antibody via thiosuccimide.

Drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker may be used to form a NDC in a simple step. In other embodiments, a bifunctional linker compound may be used to form a NDC in a two-step or multi-step process. For example, a cysteine residue is reacted with the reactive moiety of a linker in a first step, and then the functional group on the linker is reacted with a drug in the subsequent step, thereby forming a NDC.

In general, the functional group on a linker is selected so that it can specifically react with the suitable reactive group on a drug moiety. As a non-limiting example, an azide-based moiety may be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair may be used for the linker, and may also be used for the drug.

Preferably, the coupling moiety of the nanobody drug conjugate NDC in the present invention that is coupled to the nanobody moiety comprises a protein degrader or an oligonucleotide drug.

Preferably, the oligonucleotide drug is a nucleic acid drug targeting small molecules, including antisense nucleic acid (ASO), small interfering RNA (siRNA), microRNA (miRNA), small activating RNA (saRNA), messenger RNA (mRNA), aptamer, ribozyme, antibody nucleic acid conjugate drug (ARC), etc.

As used herein, the protein degrader is a degrading agent for tumor associated proteins selected from the group consisting of:
(1) kinases such as RIPK2, BCR-ABL, EGFR, HER2, c-Met, TBK1, CDK2/4/6/9, ALK, Akt, CK2, ERK1/2, FLT3, PI3K, BTK, Fak, etc;
(2) BET proteins, such as BRD2/4/6/9;
(3) nuclear receptors such as AR, ER, etc;
(4) other proteins, such as MetAp-2, Bcl-xL, Sirt2, HDAC6, Pirin, SMAD3, ARNT, PCAF/GCN5, Tau, FRS2, RAS (KRAS, HRAS, and NRAS), etc.

Preferably, the protein degrader comprises PROTAC (Protein Degradation Targeting Chimera), and more preferably, the PROTAC targets EGFR, KRAS (e.g. KRAS-G12C, KRAS-G12D).

The present invention further provides a method for preparing an ADC, which may further comprise: under a condition sufficient to form an antibody-drug conjugate (ADC), binding an antibody to a drug-linker compound.

In certain embodiments, the method according to the present invention comprises: under a condition sufficient to form an antibody-linker conjugate, binding an antibody to a bifunctional linker compound. In these embodiments, the method according to the present invention further comprises: under a condition sufficient to covalently link the drug moiety to the antibody via a linker, binding the antibody-linker conjugate to the drug moiety.

In some embodiments, an antibody-drug conjugate (ADC) has a formula as follows: 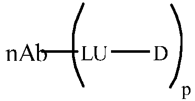 wherein:
nAb is a nanobody,
LU is a linker/connector;
D is a drug;
and the subscript p is a value selected from1 to 8.

### Drug

As used herein, the term "drug" refers to any compound possessing a desired biological activity and a reactive functional group available for preparing the conjugate of the invention. The desired biological activity includes activity useful in the diagnosis, cure, mitigation, treatment, or prevention of a disease in human or other animal. Thus, so long as it has the needed reactive functional group, the compound involved by the term "drug" include drugs identified in the official national pharmacopeia as well as e.g., official Homeopathic Pharmacopeia of the United States, or official National Formulary, or any supplements thereof. Exemplary drugs are set forth in the Physician's Desk Reference (PDR) and in the Orange Book maintained by the U.S. Food and Drug Administration (FDA). It should be understood that, as new drugs are continually discovered and developed, these drugs shall also be incorporated into the "drug" of the drug conjugates of the present invention.

The drugs useful to constitute the NDC of the present invention include, but are not limited to, cytotoxic agents (such as small molecule cytotoxic drugs).

The term "cytotoxic agents" refer to substances that inhibit or block cell expression activity, cell function and/or result in cell destruction. The term includes radioisotopes, chemotherapeutics, and toxins, such as small-molecular toxins or enzymatically active toxins (including fragments and/or variants thereof) derived from bacteria, fungi, plants or animals. Examples of cytotoxic agents include, but are not limited to: auristatins (for example, auristatin E, auristatin F, MMAE and MMAF), chlortetracycline, metotanol, ricin, ricin A-chain, cobustatin, dokamicin, dorastatin, adriamycin, daunorubicin, paclitaxel, cisplatin, cc1065, ethidium bromide, mitomycin, etoposide, tenoposide, vincristine, vinblastine, colchicine, dihydroxyanthracnose diketone, actinomycin, diphtheria toxin, Pseudomonas exotoxin (PE) A, PE40, abrin, abrin A chain, modeccin A chain, α-Sarcina, gelonin, mitogellin, retstrictocin, phenomycin, enomycin, curicin, crocotin, calicheamicins, Sapaonaria officinalis inhibitor, as well as glucocorticoid and other chemotherapy agents, as well as radioisotopes such as At²¹¹, I¹³¹, I¹²⁵ , Y⁹⁰ , Re¹⁸⁶, Re¹⁸⁸ , Sm¹⁵³ , Bi²¹² or Bi²¹³, P³² and Lu (including Lu¹⁷⁷). Antibodies can also be conjugated to anticancer prodrug activating enzymes that can convert a prodrug into the active form.

One preferred drug useful in the present invention is maytansine or maytansinoids. Maytansine inhibits cell proliferation by inhibiting the formation of microtubules from tubulins. Maytansinoids are derivatives of maytansine. Both maytansine and maytansinoids are highly cytotoxic, but they are greatly limited for clinical use in cancer therapy due to poor selectivity for tumors. However, a high cytotoxicity enables them to be attractive drug moieties in ADCs. The structure shown below is deacetyl-maytansine.

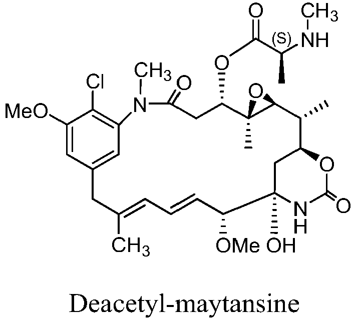

Another preferred drug useful in the present invention is auristatins. Auristatins are synthetic analogues of dolastatin 10, which is a polypeptide isolated from marine mollusk Aplysia having biological activity. Dolastatin 10 inhibits tubulin polymerization by binding to tubulin at the same domain as anticancer drug vincristine. dolastitin 10, auristatin PE, and auristatin E are all linear peptides having four amino acids, three of which are unique to the dolastatin class compounds, and a C-terminal amide group. Two representative auristatins, monomethyl auristatin E (MMAE) and monomethyl auristatin F (MMAF), are preferred drug moiety candidates for ADCs.

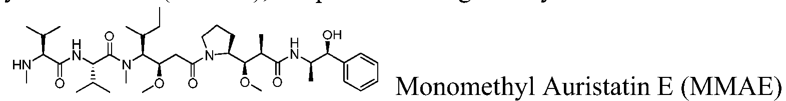

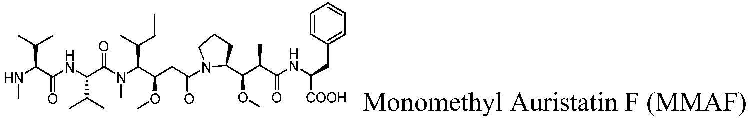

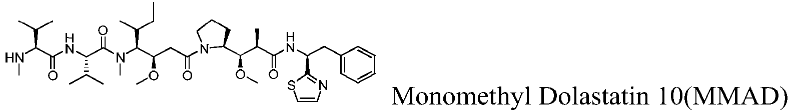

Another preferred drug of the present invention is microtubuleysin (tubulysin). Microtubuleysin was first isolated by the research team from slime cell cultures and is a highly effective cell growth inhibitor that works by inhibiting microtubule protein polymerization and inducing cell apoptosis. Microtubuleysin D in microtubuleysins is the most effective, with activity 10 to 100 times higher than most other microtubule protein regulators, including erythromycin, vinblastine, and paclitaxel. Paclitaxel and vinblastine are currently used for the treatment of various cancers, while epomycin derivatives are undergoing activity evaluation in clinical trials. The synthetic derivatives of microtubuleysin D will provide necessary information related to inhibition and critical binding interactions, and can exhibit superior properties as anticancer agents, either as isolated entities or as chemical warheads on targeted antibodies or ligands. Microtubuleysin D is a complex tetrapeptide that may be divided into four regions: Mep (D-N-methylpiperidinecarboxylic acid), Ile (isoleucine), Tuv (tubulvaline), and Tup (tubulphenylalanine), as shown in the following formula:

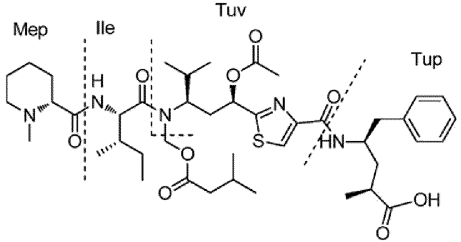

Another preferred drug of the present invention is a cryptophycin derivative derived from microorganisms that can inhibit microtubule polymerization. Cryptophycin is a novel anti-tumor active substance isolated from blue-green algae cultures that can inhibit microtubule formation and has activity against various tumors. Cryptophycin is a lipophilic compound containing 2 peptide bonds and 2 ester bonds, with 5 optically active centers and 1 epoxy group. The dipeptide diester bonds are all in a macrocyclic structure. The structures of Cryptophycin derivatives CP1 and CP2 are shown in the following formulas:

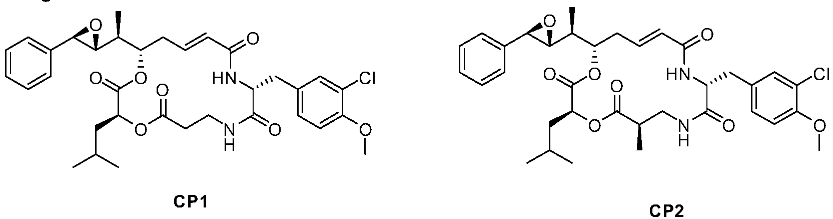

Another preferred drug of the present invention is the novel microtubule inhibitor Taltobulin (HTI-286, SPA-110). Taltobulin inhibits the polymerization of purified microtubules, interferes with intracellular microtubule organization, induces mitotic blockade, and induces cell apoptosis. Taltobulin is a potent inhibitor of cell proliferation, with an average IC50 of 2.5 nM against 18 human tumor cell lines. Compared with the currently used microtubule inhibitors, Taltobulin is not a suitable substrate for p-glycoprotein, and its structure is shown in the following figure:

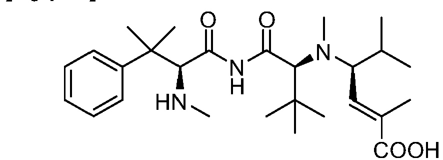

On the one hand, the drug is a derivative of camptothecin, SN-38. SN-38 is a bioactive metabolite of irinotecan hydrochloride (CPT-11) and a class of topoisomerase inhibitors. SN-38 causes the strongest inhibition of DNA topoisomerase I, dose- and time-dependent inhibition of DNA synthesis, and frequent DNA single strand breaks. The structure of SN-38 is shown in the following figure:

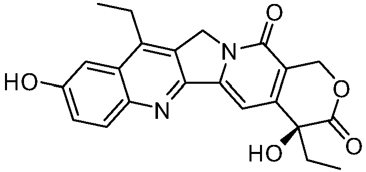

On the other hand, the drug is Exatecan, a derivative of camptothecin. It is a synthetic analogue of the topoisomerase I inhibitor camptothecin, with stronger activity than SN-38. It can cause the strongest inhibition of DNA topoisomerase I, dose- and time-dependent inhibition of DNA synthesis, and frequent DNA single strand breaks. The Exatecan structure is shown in the following formula:

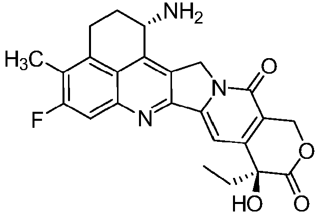

Another preferred drug of the present invention is alpha-Amanitin, which has the structure shown in the following figure. Alpha-Amanitin is a fungal toxin derived from Amanita phalloides, a poisonous mushroom. It has a bicyclic octapeptide and can inhibit the transcription of eukaryotic RNA polymerase II and RNA polymerase III.

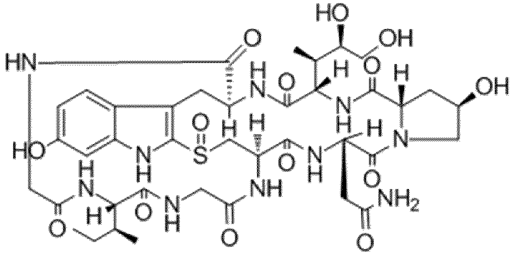

Another preferred drug of the present invention is a benzodiazepine antibiotic (duocarmycins, CC-1065, etc.) and other cyclopropyrrolidind-4-one (CPI) derivatives. This type of compound is an effective DNA small groove binding alkylating reagent. Cyclopropabenzindol-4-one (CBI) analogues have more stable chemical structures, higher biological activity, and are easier to synthesize compared to their parent compounds containing natural CPI alkylated subunits. A representative CBI derivative is the phenolic hydroxyl protected derivative CBI, which has weakened prodrug toxicity and enhanced water solubility (the CBI*-seco* class structural formula is shown in the following figure):

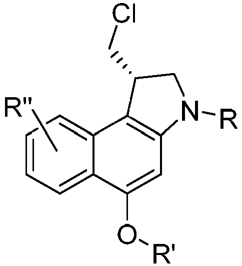

Another preferred drug of the present invention is pyrrolo[2,1-c][1,4]benzodi-azepine (PBDs) or PBD dimers. PBD is a natural product produced by Streptomyces, characterized by the ability to form non twisted covalent adducts in DNA grooves, specifically at the purine-guanine-purine sequence. The application of PBD as a partial small molecule strategy to target DNA sequences and as a novel anti-cancer and antibacterial drug has attracted increasing interest. The application of a flexible carbon chain to connect the C8/C8' hydroxyl groups of two PBD units results in a dimer with enhanced biological activity. PBD dimers are believed to generate sequence selective DNA damage, such as reverse 5'-Pu-GATC-Py-3' interchain cross-linking, leading to their biological activity. These compounds have been proven to be highly effective cytotoxic drugs and can serve as alternative drugs for antibody drug conjugates.

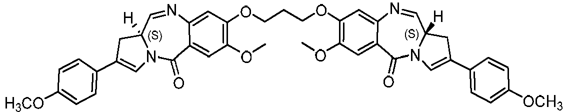

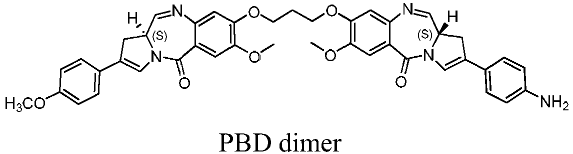

Another preferred drug of the present invention is a derivative of PNU-159682, which is the main active metabolite of Nemorubicin in human liver microsomes. Compared with MMDX and doxorubicin, PNU-159682 exhibits a 3000 folds increase in activity.

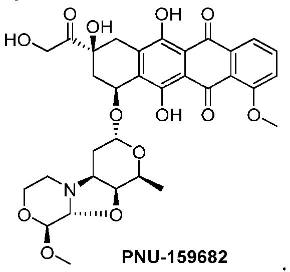

On the other hand, drugs are not limited to the categories mentioned above, but also include all drugs that may be used for antibody drug conjugates. And especially those that can coordinate through amide bonds with the linker, such as cell toxins that coordinate through basic amino groups (primary or secondary amines), such as the structure of cell toxins D1-D12 shown above.

### Linker

According to the mechanism of drug release within cells, "linker" or "linker of antibody drug conjugate" may be divided into two categories: unbreakable linker and breakable linker.

For antibody drug conjugates containing unbreakable linker, the drug release mechanism is as follows: after the conjugate binds to the antigen and is internalized by the cell, the antibody is enzymatically hydrolyzed in the lysosome, releasing an active molecule composed of small molecule drugs, linkers, and antibody amino acid residues. The resulting changes in the molecular structure of the drug do not weaken its cytotoxicity, but due to the charged nature of the active molecule (amino acid residues), it cannot penetrate neighboring cells. Therefore, such active drugs cannot kill adjacent tumor cells that do not express the targeted antigen (antigen negative cells) (bystander effect).

The breakable linker, as the name suggests, can break and release active drugs (small molecule drugs themselves) within the target cell. Breakable linkers may be divided into two main categories: chemically unstable linkers and enzymatically unstable linkers. Chemical unstable linkers can selectively break due to differences in plasma and cytoplasmic properties. Such properties include pH value, glutathione concentration, etc. The pH sensitive linkers are commonly referred to as acid break linkers. Such linkers are relatively stable in the neutral environment of blood (pH 7.3-7.5), but will be hydrolyzed in weakly acidic endosomes (pH 5.0-6.5) and lysosomes (pH 4.5-5.0). The first generation of antibody drug conjugates mostly used such linkers, such as hydrazones, carbonates, acetals, and ketones. Due to the limited plasma stability of acid cleaved linkers, antibody drug conjugates based on such linkers typically have a short half-life (2-3 days). This relatively short half-life to some extent limits the application of pH sensitive linkers in the new generation of antibody drug conjugates.

For glutathione sensitive linker, it is also known as disulfide bond linkers. Drug release is based on the difference between the high concentration of intracellular glutathione (in the millimolar range) and the relatively low concentration of glutathione in the blood (in the micromolar range). This is especially true for tumor cells, whose low oxygen content leads to increased activity of reductases, resulting in higher concentrations of glutathione. Disulfide bond has thermodynamic stability and therefore exhibits good stability in plasma.

Enzyme unstable linker, such as peptide linkers, can better control drug release. Peptide linkers may be effectively cleaved by proteases within the lysosome, such as cathepsin B or fibrinolytic enzymes (which have increased levels in some tumor tissues). This peptide linkage is considered to be very stable in plasma circulation, as inappropriate pH values outside the cell and serum protease inhibitors often render proteases inactive. Due to its high plasma stability and good intracellular cleavage selectivity and effectiveness, enzyme unstable linkers are widely used as breakable linkers for antibody drug conjugates. Typical enzyme unstable linkers include Val-Kit (VC), Val-Ala (VA), Gly-Gly-Phe-Gly (GGFG), etc.

The self-releasing linker is generally embedded between the breakable linker and the active drug, or is itself a part of the breakable linker. The mechanism of action of the self-releasing linker is that when the breakable linker is broken under appropriate conditions, the self-releasing linker can spontaneously undergo structural rearrangement, thereby releasing the active drug connected to it. Common suicide linkers include para aminobenzyl alcohol (PAB) and β-glucuronide, etc.

### Application

The present invention also provides use of the antibodies of the present invention, such as for preparing diagnostic preparations, or for preparing medicine for preventing and/or treating CD73-related diseases. The CD73-related diseases include tumor occurrence, growth and/or metastasis, tumor resistance related diseases, inflammation, metabolism-related diseases, etc.

Use of the antibodies, NDCs or CAR-Ts of the present invention includes (but is not limited to):
(i) diagnosis, prevention and/or treatment of tumorigenesis, tumor growth and/or metastasis, particularly, for a tumor with a CD73 high expression; wherein the tumor includes (but is not limited to): breast cancer (e.g. triple negative breast cancer), lung cancer (such as non-small cell lung cancer), pancreatic cancer, malignant glioma, gastric cancer, liver cancer, esophageal cancer, kidney cancer, colorectal cancer, bladder cancer, prostate cancer, endometrial cancer, ovarian cancer, cervical cancer, leukemia, bone marrow cancer, angiosarcoma, etc.; preferably triple negative breast cancer, non-small cell lung cancer, pancreatic cancer, malignant glioma; and more preferably triple negative breast cancer and/or non-small cell lung cancer;
(ii) diagnosis, prevention and/or treatment of an autoimmune disease; wherein the autoimmune disease includes (but is not limited to): systemic lupus erythematosus, rheumatoid arthritis, ulcerative colitis, type I diabetes, psoriasis, multiple sclerosis;
(iii) diagnosis, prevention and/or treatment of inflammation; wherein the inflammation includes (but is not limited to): rheumatic arthritis, osteoarthritis, ankylosing spondylitis, gout, Lytle syndrome, psoriasis arthritis, infectious arthritis, tuberculous arthritis, viral arthritis, fungal arthritis, glomerular Nephritis, systemic lupus erythematosus, Crohn's disease, ulcerative colitis, acute lung injury, chronic obstructive pulmonary disease, and idiopathic pulmonary fibrosis;
(iv) diagnosis, prevention and/or treatment of a metabolism-related disease, wherein the metabolism-related disease includes (but is not limited to): diabetes, diet-induced obesity, adipose inflammation.

### Pharmaceutical composition

The invention further provides a composition. In the preferred examples, the composition is a pharmaceutical composition comprising the antibody, or an active fragment, a fusion protein or a NDC thereof, or a corresponding CAR-T cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, although the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention can be directly used for binding to CD73 protein molecule, and thus can be used for preventing and treating diseases such as tumors. In addition, other therapeutic agents can also be used simultaneously.

The pharmaceutical composition according to the present invention comprises a safe and effective amount (e.g. 0.001-99 wt%, preferably 0.01-90 wt%, preferably 0.1-80 wt%) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 µg/kg body weight to about 5 mg/kg body weight per day. Further, the polypeptide of the present invention can also be used in combination with the other therapeutic agents.

When a pharmaceutical composition is used, a safe and effective amount of the immunoconjugate is administered to a mammal, wherein the safe and effective amount is usually at least about 10 µg/kg body weight, and in most cases does not exceed about 50 mg/kg body weight, preferably the dose is about 10 µg/kg body weight to about 20 mg/kg body weight. Of course, the particular dose should also depend on various factors, such as the route of administration, patient healthy status, which are well within the skills of an experienced physician.

As for NDC, the nanobody-drug conjugate provided by the present invention can target a specific cell population and bind to a specific protein (antigen) on cell surface, thereby releasing the drug into the cell in an active form through endocytosis or drug infiltration of the conjugate. Thus, the nanobody-drug conjugate of the invention can be used to treat diseases of interest, and the antibody-drug conjugate mentioned above can be administered to a subject (e.g., a human) by a suitable route in a therapeutically effective amount. A subject in need of treatment can be a patient at risk of having or suspected of having a condition associated with the activity or amount of expression of a particular antigen. Such patients can be identified by routine physical examination.

When the nanobody-drug conjugate as described herein is used as the therapeutic agent, it can be delivered by methods conventional in the art. For example, it can be introduced into cells using liposomes, hydrogels, cyclodextrins, biodegradable nanocapsules, or bioadhesive microspheres. Alternatively, nucleic acids or vectors can be delivered *in situ* by direct injection or by use of an infusion pump.

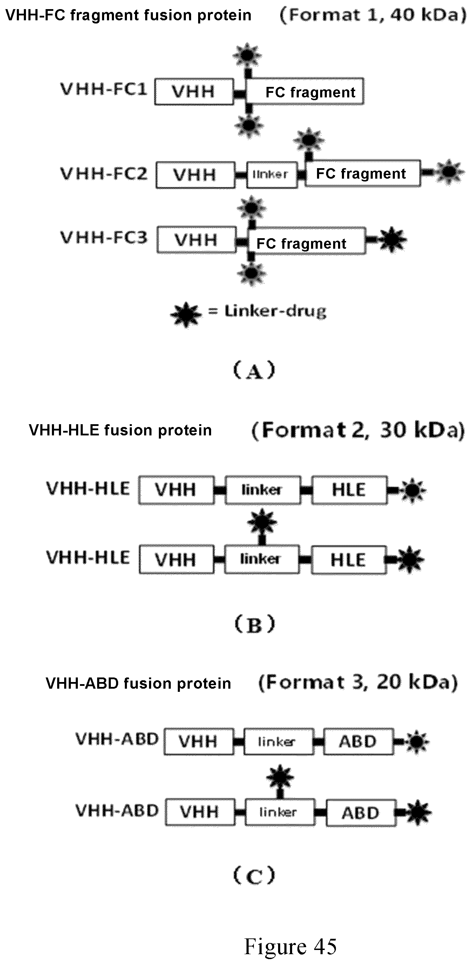

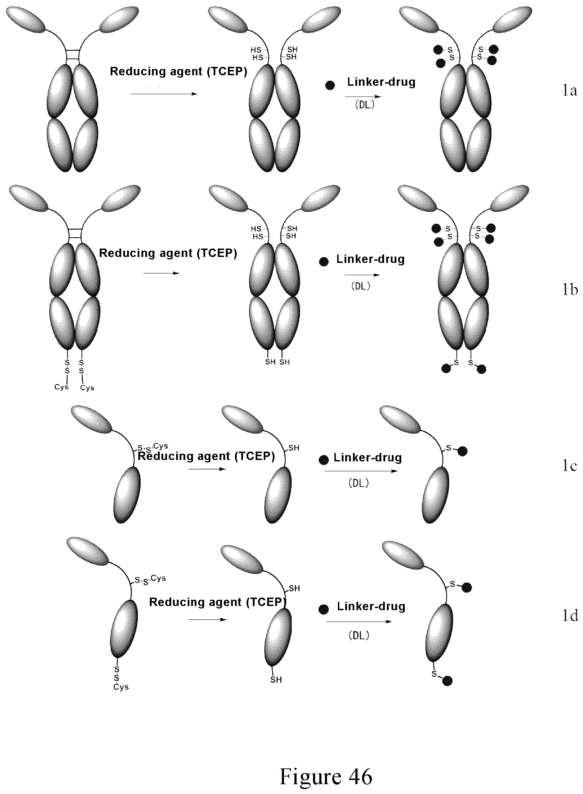

The main advantages of the present invention include:
1. The nanobody of the present invention has excellent biological activity and specificity, and has a very high affinity (EC₃₀ ranges from 2.67-13.48 ng/mL as determined by ELISA), and has inhibitory activity against the CD73 enzyme function (IC₅₀ ranges from 4.66-38.18 ng/mL as determined by enzyme activity). In addition, it has excellent binding affinity to CD73 of tumor cells (EC₅₀ ranges from 0.037-0.227 µg/mL as determined by FACS), and inhibits the function of CD73 enzyme of tumor cells (IC₅₀ value ranges from 0.083-0.48 µg/mL), and can be used as a therapeutic antibody targeting CD73.
2. The nanobody and nanobody-drug conjugates (NDC) of the present invention have significant anti-tumor activity and have no obvious toxic side effects on normal cells.
3. The nanobody of the present invention has a significant protective effect on human T lymphocytes proliferation, can effectively reverse the proliferation inhibition of T lymphocytes by adenosine monophosphate (AMP), and promote the expression and secretion of INFγ, and its EC₅₀ ranges from 0.0008-0.0247 µg/mL.
4. According to the design of Figure 45 a, b, and c of the present invention, CD73-nanobody-drug conjugates with desired DAR values can be easily prepared, and their cross-linking reaction formulas are shown in Figures 46 1a, 1b, 1c, or 1d, respectively.
5. The nanobody-drug conjugate (NDC) of the present invention has excellent CD73-dependent anti-tumor activity, which has no obvious toxic side effects on cells with a normal or low expression of CD73, and has extremely high killing activity on tumor cells with a medium or high expression of CD73. Its IC₅₀ ranges from 0.0025-0.0658 µg/mL as determined by cell proliferation inhibition test.
6. In the intermittent dosing model, NDC showed stronger anti-tumor killing effect than ordinary antibody-drug conjugates (ADCs) against the same target.
7. The NDC of the present invention has no obvious toxic side effects on the proliferation of normal human T lymphocytes, and its IC₅₀ is >15 µg/mL as determined by a cell proliferation inhibition test.
8. The NDC of the present invention has a significant protective effect on human T lymphocytes proliferation, can effectively reverse the proliferation inhibition of T lymphocytes by adenosine monophosphate (AMP) and promote the expression and secretion of INFγ, and its EC₅₀ ranges from 0.0046-0.0379 µg/mL.
9. The NDC of the present invention has extremely excellent *in vivo* anti-tumor activity. After a single intravenous injection, it can completely inhibit tumor growth or cause tumor regression in multiple tumor models.
10. The nanobody and NDC of the present invention can penetrate the blood-brain barrier, have the characteristics of distribution in the brain, and show excellent therapeutic effects on intracranial tumors.
11. The humanized antibody and NDC of the present invention also exhibit the above advantages or better.

The present invention will be further illustrated below with reference to the specific examples. It is to be understood that these examples are for illustrative purposes only and are not intended to limit the scope of the invention. For the experimental methods in the following examples, in which the specific conditions are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless otherwise specified, percentages and parts are calculated by weight.

### Example 1 Discovery and preparation of nanobodies targeting human CD73

Firstly, the extracellular domain of human CD73 protein (CD73-ECD) was prepared as an antigen. By referring to NCBI: NP_002517.1 amino acid at positions 27 to 547, C-terminus polyhistidine-tagged antigen was obtained using gene cloning technology and mammalian vector expression system:

WELTILHTNDVHSRLEQTSEDSSKCVNASRCMGGVARLFTKVQQIRRAEP
NVLLLDAGDQYQGTIWFTVYKGAEVAHFMNALRYDAMALGNHEFDNGVEGL
IEPLLKEAKFPILSANIKAKGPLASQISGLYLPYKVLPVGDEVVGIVGYTSKETPF
LSNPGTNLVFEDEITALQPEVDKLKTLNVNKIIALGHSGFEMDKLIAQKVRGVD
VVVGGHSNTFLYTGNPPSKEVPAGKYPFIVTSDDGRKVPVVQAYAFGKYLGYL
KIEFDERGNVISSHGNPILLNSSIPEDPSIKADINKWRIKLDNYSTQELGKTIVYLD
GSSQSCRFRECNMGNLICDAMINNNLRHTDEMFWNHVSMCILNGGGIRSPIDER
NNGTITWENLAAVLPFGGTFDLVQLKGSTLKKAFEHSVHRYGQSTGEFLQVGGI
HVVYDLSRKPGDRVVKLDVLCTKCRVPSYDPLKMDEVYKVILPNFLANGGDG
FQMIKDELLRHDSGDQDINVVSTYISKMKVIYPAVEGRIKAHHHHHHHHH

Alpaca was immunized with the CD73 extracellular domain protein prepared above, with an interval of 21 days. Part of peripheral blood (PBMC) was collected 10 days after the last immunization. After PBMC separation and VHH antibody fragment cloning, phage display library and yeast display library were constructed. Through repeated panning, ELISA of monoclones, FACS screening and other steps, 4 highly active nanobodies (3-D7, 5-E11, 4-D04, 4-B02) with independent sequences were obtained from the phage display library, and 5 active antibodies (1-E7, 2-B10, 2-G9, 3-A3, 3-F1) with independent sequences were obtained from the yeast display library.

### Example 2 SPR assay of affinity of CD73 nanobodies to human CD73 antigen

Surface plasmon resonance (SPR) assay: Protein A sensor was used to immobilize the antibodies, with an immobilization height of about 1.5 nM. The buffer was PBST (PBS + 0.02% tween-20), and the CD73-ECD protein sample was diluted to 500, 250, 125, 62.5, 31.3, 0 nM. Affinity detection: equilibrium for 60 s, binding for 180 s, dissociation for 180 s, at a detection temperature of 25 °C. Kinetic characterization analysis was performed using the ForteBio OCTET R2 system.

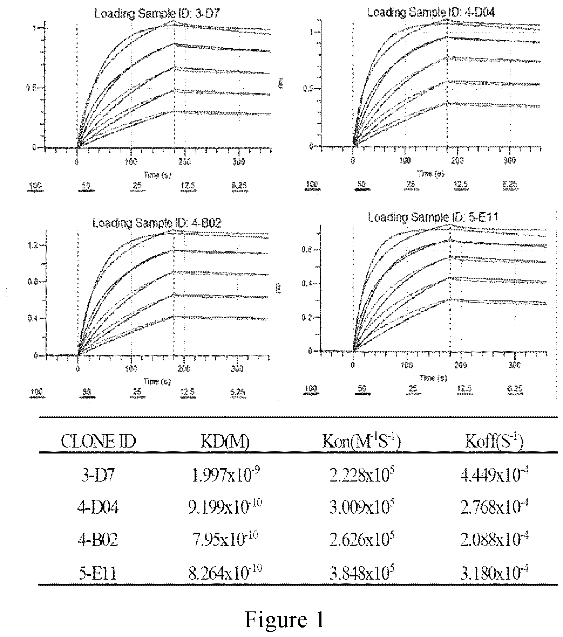

The detection results are shown in Figure 1. 3-D7, 5-E11, 4-D04, and 4-B02 have strong affinity to CD73-ECD, with the binding constants (KD) of 1.997 nM, 0.826 nM, 0.919 nM, and 0.795 nM, respectively.

### Example 3 ELISA determination of affinity of CD73 nanobodies to human CD73 antigen

The CD73 protein extracellular region (CD73-ECD) was diluted to 1 µg/mL using the coating solution, and an ELISA plate was coated at 100 µL/well at 4°C overnight. Excess antigens were washed away, and the plate was blocked with 1% BSA at room temperature for 2 hours. Then, 5-fold gradient dilutions of each monoclonal antibody were added at 100 µL/well and incubated at room temperature for 1 hour. Unbound antibodies were washed away, and an appropriate concentration of Staphylococcal protein A labeled with horseradish peroxidase was added at 100 µL/well and incubated at room temperature for 0.5 hours. Unbound secondary antibodies were washed away, and TMB color development solution was added to react for about 15 minutes. 1M HCL was added at 50 µL/well to terminate the color development reaction, and then the absorbance was measured at 450 nm and the data was analyzed.

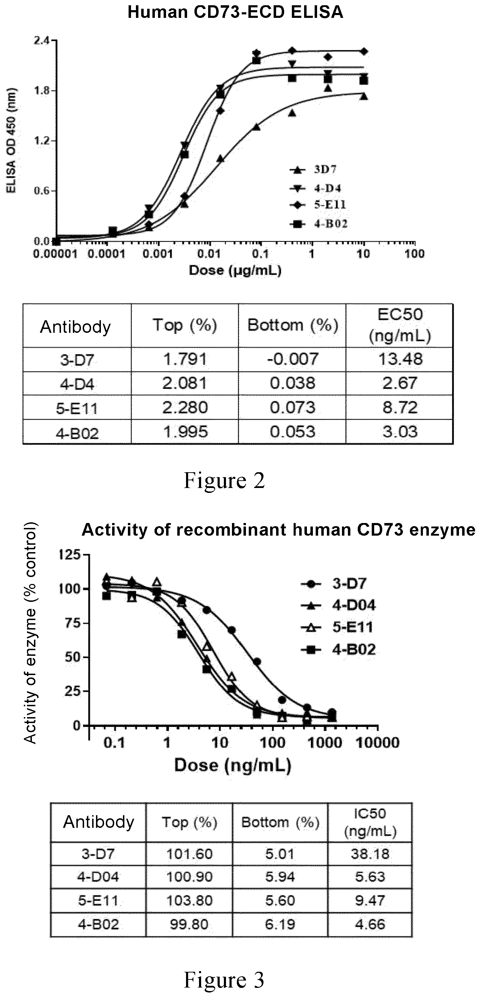

The test results are shown in Figure 2. 3-D7, 5-E11, 4-D04, and 4-B02 have a strong affinity to CD73-ECD, with EC₅₀ of 13.48 ng/mL, 8.72 ng/mL, 2.67 ng/mL, and 3.03 ng/mL, respectively.

For the five antibodies from yeast, after transient expression in HEK293F cells, the supernatant was collected after 2 days of continuous culture and ELISA assay was performed using 100 µL, 10 µL, and 1 µL of the supernatant.

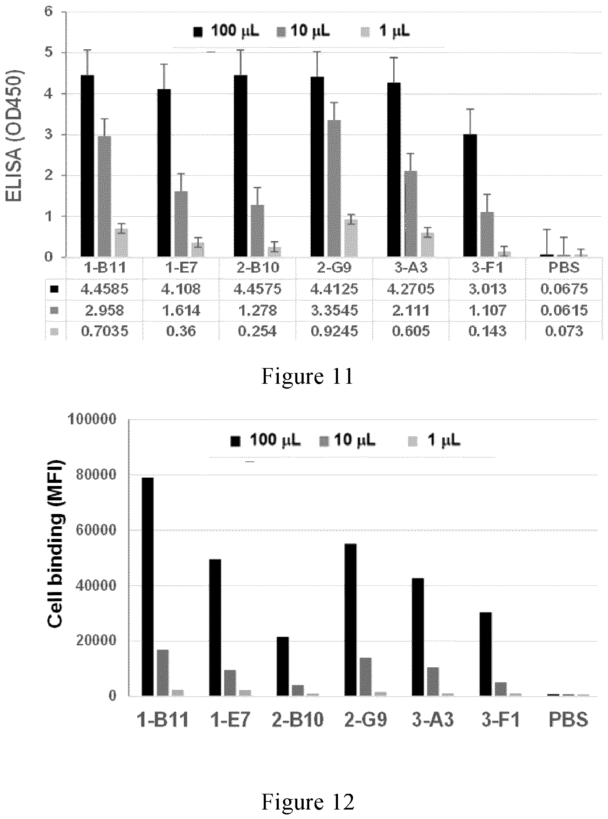

The test results are shown in Figure 11. Nanobodies 1-E7, 2-B10, 2-G9, 3-A3, and 3-F1 all had strong CD73-ECD binding affinity.

The above results show that the CD73 nanobodies in this example have good binding activity to the human CD73 antigen.

### Example 4 Determination of the inhibitory activity of CD73 nanobodies to the catalytic function of recombinant human CD73 enzyme

The human recombinant CD73 enzyme (CD73 extracellular region) was diluted to 0.1 µg/mL with antigen diluent and was evenly plated in a 96-well low-adsorption culture plate at 25 µL/well. 50 µL of CD73 antibody diluted from 300 ng/mL to 0.137 ng/mL in a 3-fold gradient was added to the culture plate, mixed (final concentration is 1350 ng/mL to 0.069 ng/mL), incubated at 37 °C for 1 hour. 25 µL of a mixture containing 1.2 mM AMP and 0.4 mM ATP was added, and incubated at 37 °C for 1 hour. 50 µL of the above reaction solution was taken out and added to another 96-well white plate. 50 µL of CellTiter-Glo reagent was added to each well, mixed and reacted in the dark for 3-5 minutes, and the intensity of the fluorescence signal was detected using a microplate reader.

The test results are shown in Figure 3. 3-D7, 5-E11, 4-D04, and 4-B02 all have the activity of significantly inhibiting the hydrolysis of AMP by recombinant CD73 protease, with IC₅₀ of 38.18 ng/mL, 9.47 ng/mL, 5.63 ng/mL, and 4.66 ng/mL, respectively.

The above results show that the CD73 monoclonal nanobodies in this example have significant inhibitory activity to the catalytic function of the recombinant human CD73 enzyme.

### Example 5 Determination of the binding affinity of CD73 nanobodies to CD73 on the surface of tumor cells

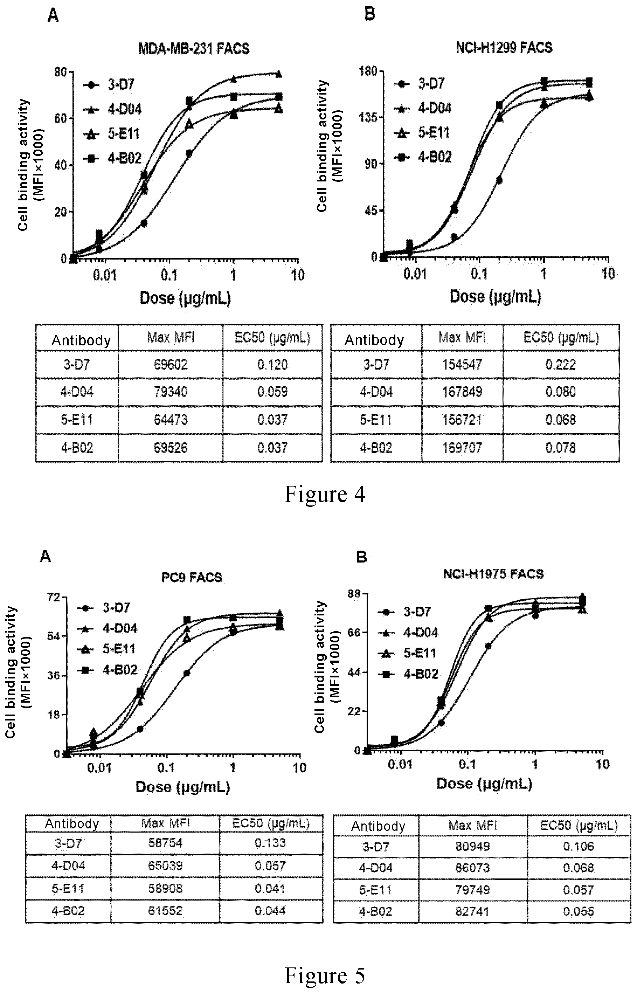

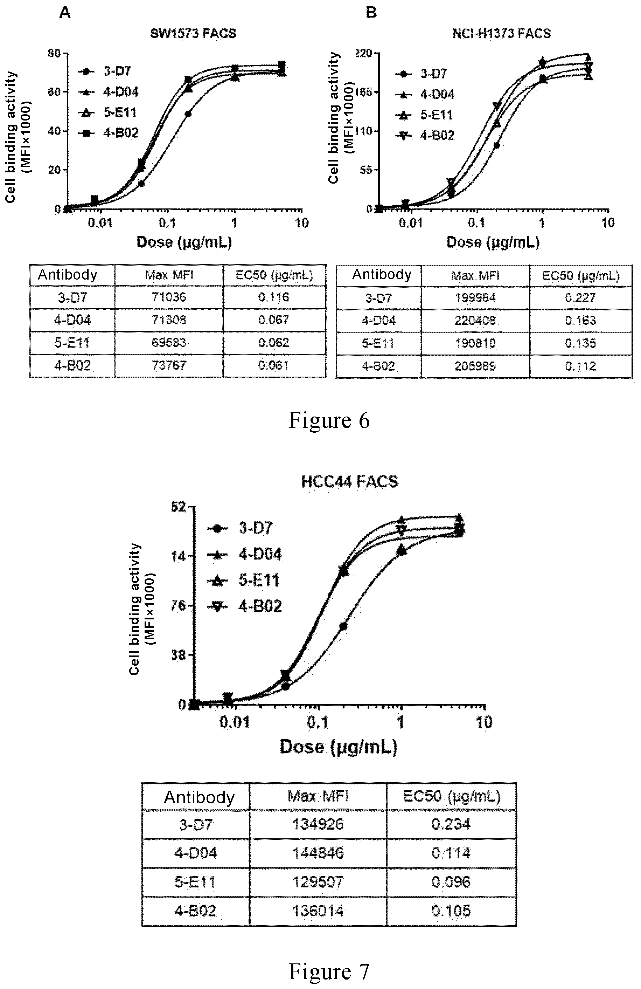

Triple-negative breast cancer cells MDA-MB-231, non-small cell lung cancer cells NCI-H1299, PC9, NCI-H1975, SW1573, NCI-H1373, and HCC44 with medium or high expression of CD73 were used as target cells. 100 µL of the test antibodies diluted from 10 µg/mL to 0.016 µg/mL in a 5-fold gradient were used as the primary antibodies, and were mixed with 1×10⁵ tumor cells suspended in 100 µL RPMI-1640 serum-free medium to make a final concentration of 5 µg/mL diluted to 0.008 µg/mL, and then incubated at 4°C for 1 h. The cells were washed twice with PBS to remove unbound primary antibodies, and then the target cells were incubated with 200 µLPE-labeled secondary antibody at 2 µg/mL at 4°C for 30 min. The cells were washed twice with PBS to remove unbound secondary antibodies, and finally the cells were resuspended in 200 µL PBS. The binding affinity of the test antibodies to CD73 on the surface of the corresponding cells was measured by flow cytometry.
The test results are shown in Figure 4A. 3-D7, 5-E11, 4-D04, and 4-B02 have excellent binding affinity to MDA-MB-231, with EC₅₀ of 0.12 µg/mL, 0.037 µg/mL, 0.059 µg/mL, and 0.037 µg/mL, respectively;
The test results are shown in Figure 4B. 3-D7, 5-E11, 4-D04, and 4-B02 have excellent binding affinity to NCI-H1299, with EC₅₀ of 0.222 µg/mL, 0.068 µg/mL, 0.080 µg/mL, and 0.078 µg/mL, respectively;
The test results are shown in Figure 5A. 3-D7, 5-E11, 4-D04, and 4-B02 have excellent binding affinity to PC9, with EC₅₀ of 0.133 µg/mL, 0.041 µg/mL, 0.057 µg/mL, and 0.044 µg/mL, respectively;
The test results are shown in Figure 5B. 3-D7, 5-E11, 4-D04, and 4-B02 have excellent binding affinity to NCI-H1975, with EC₅₀ of 0.106 µg/mL, 0.057 µg/mL, 0.068 µg/mL, and 0.055 µg/mL, respectively;
The test results are shown in Figure 6A. 3-D7, 5-E11, 4-D04, and 4-B02 have excellent binding affinity to SW1573, with EC₅₀ of 0.116 µg/mL, 0.062 µg/mL, 0.067 µg/mL, and 0.061 µg/mL, respectively;
The test results are shown in Figure 6B. 3-D7, 5-E11, 4-D04, and 4-B02 have excellent binding affinity to NCI-H1373, with EC₅₀ of 0.227 µg/mL, 0.135 µg/mL, 0.163 µg/mL, and 0.112 µg/mL, respectively;
The test results are shown in Figure 7. 3-D7, 5-E11, 4-D04, and 4-B02 have excellent binding affinity to HCC44, with EC₅₀ of 0.234 µg/mL, 0.096 µg/mL, 0.114 µg/mL, and 0.105 µg/mL, respectively;

For the six antibodies from yeast, after transient expression in HEK293F cells, the supernatant was collected after 2 days of continuous culture. The binding activity was measured by flow cytometry using 100 µL, 10 µL, and 1 µL of the supernatant that incubated with MDA-MB-231 cells.

The test results are shown in Figure 12. Nanobodies 1-E7, 2-B10, 2-G9, 3-A3, and 3-F1 all have strong CD73-ECD binding affinity.

The above results show that the CD73 nanobodies of this example can target CD73 of human tumor cells and have excellent binding activity.

### Example 6 Effect of CD73 nanobody on the catalytic function of CD73 enzyme on the surface of tumor cells

Triple-negative breast cancer cell MDA-MB-231 with CD73 high expression was used as a target cell. An appropriate number of tumor cells (confirmed by preliminary experiments) were plated in a 96-well plate, and cultured at 37 °C for 16 hours, then the cells were washed three times with serum-free RPMI-1640 medium, and 50 µL of the test antibody diluted in 3-fold gradient from 10 µg/mL to 0.0045 µg/mL was added to the 96-well plate. After incubation at 37 °C for 30 minutes, 25 µL of 0.9 mM AMP was added and cultured at 37 °C, 5% CO₂ for 3 hours. 25 µL of the above culture supernatant was taken out and added to another 96-well white plate. 25 µL of 0.1 mM ATP was added, and mixed. 50 µL of CellTiter-Glo reagent was added to each well, mixed and reacted in the dark for 3 to 5 minutes. The fluorescence signal intensity was detected by a microplate reader.

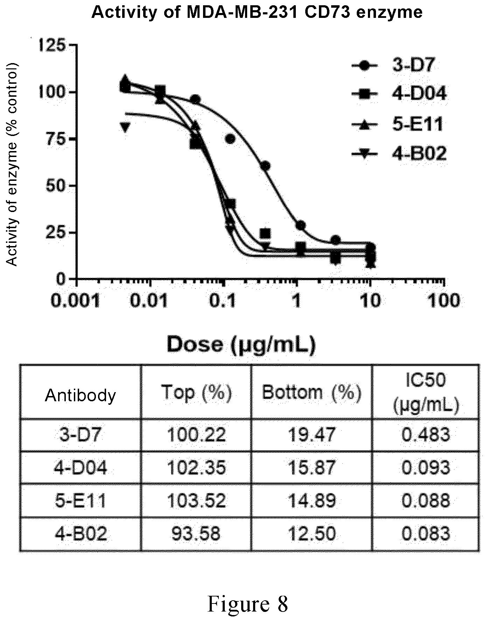

The test results are shown in Figure 8. 3-D7, 5-E11, 4-D04, and 4-B02 can all significantly inhibit the function of CD73 on the surface of MDA-MB-231 cells to catalyze the hydrolysis of AMP, with IC₅₀ of 0.483 µg/mL, 0.088 µg/mL, 0.093 µg/mL, and 0.083 µg/mL, respectively.

### Example 7 Protective effect of CD73 nanobodies on T lymphocyte proliferation and IFN-γ expression

Recovery, expansion and sorting of PBMC: firstly, PBMC were recovered and cultured for 3-4 days in a culture medium containing 500 ng/mL CD3/CD28 antibody and 100 IU/mL IL-2. Then, PBMC were sorted using a sorting kit (Stemcell, Cat#1795) to obtain CD3-positive T lymphocytes.

T lymphocyte proliferation test: the T lymphocytes obtained by the above sorting were fluorescently labeled, and the pre-prepared CFSE (carboxyfluorescein succinimidyl ester) was added to the cell suspension (final concentration of 2.5 µM), labeled at 37 °C for 5 minutes, and then washed three times with PBS. Then, the CFSE-labeled T cells were plated in a 96-well plate (2x10⁴ cells/well), and 50 µL of nanobodies diluted in a gradient (final concentration of 15 µg/mL to 0.00019 µg/mL, n=4) were added to each well, and 50 µL of adenosine monophosphate (AMP, final concentration of 0.25 mM) was added, mixed, and cultured for 4-5 days. The culture supernatant was collected, and the number of cells in a fixed volume was read and counted using a flow cytometer (FACS). The cell proliferation curve was drawn using Flowjo software and the EC₅₀ values were calculated. 50µL/well of T cell culture supernatant was taken to detect the concentration of IFN-γ protein using an ELISA kit and by referring to the technical operation steps provided in the kit.

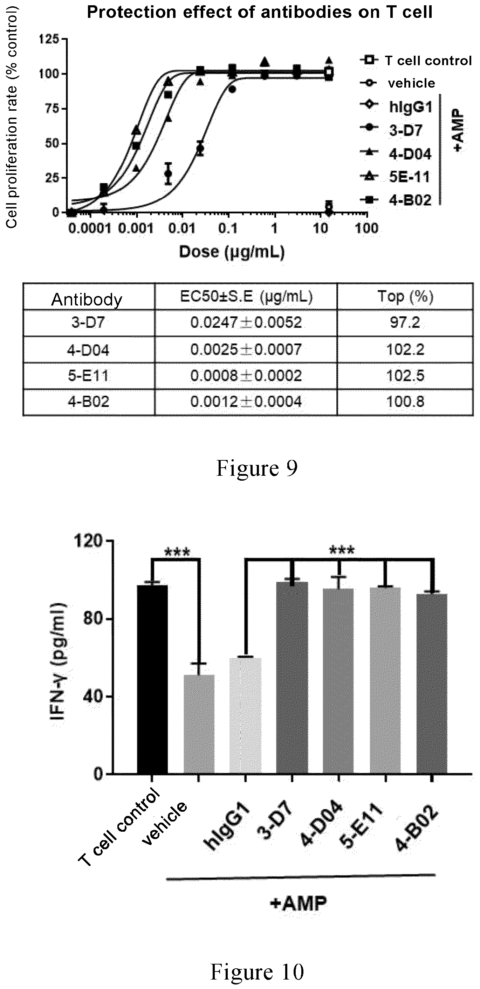

The results are shown in Figure 9. CD73 nanobodies 3-D7, 5-E11, 4-D04, and 4-B02 have significant proliferation protective effects on human T lymphocytes and can effectively reverse the proliferation inhibitory effect of AMP on T lymphocytes. Their EC₅₀ values are 0.0247±0.0052 µg/mL, 0.0008±0.0002 µg/mL, 0.0025±0.0007 µg/mL, and 0.0012±0.0004 µg/mL, respectively.

The results are shown in Figure 10. CD73 nanobodies 3-D7, 5-E11, 4-D04, and 4-B02 can effectively reverse the inhibitory effect of AMP on the expression/secretion of INF-γ.

The above results show that the CD73 nanobody in this example can not only inhibit the catalytic function of CD73 enzyme on the surface of tumor cells, but also inhibit the catalytic function of CD73 enzyme on the surface of T cells, reverse the effect of AMP on the proliferation and function of T cells, therefore protect T cells.

### Example 8 CD73 antibodies bind to tumor cells, resulting in internalization into intracellular lysosomes

MDA-MB-231 cells were plated in a 26-well plate at 50% density and cultured at 37 °C for 16 h. 5 µg/mL CD73 nanobodies were added and incubated at 37 °C for 4 h or 4 °C for 1 h. The cells were washed three times with PBS to remove the antibodies that were not bound to the cells and fixed with 4% paraformaldehyde at room temperature for 30 min. The cells were washed three times with PBS and permeabilized with 0.4% Triton X-100 for 10 min. After washed three times with PBS, Lamp-2 (rabbit anti-human) antibodies were incubated at 37°C for 1 h to mark the location of lysosomes in cell. Unbound antibodies were washed with PBS, and R-PE-labeled goat anti-human and Alexa Fluor 488-labeled donkey anti-rabbit secondary antibodies were incubated at 37°C for 30 min. Unbound secondary antibodies were washed away, and DAPI was used for staining for 10 min to mark the location of the cell nucleus, then antibody internalization was observed with a fluorescence microscope (200×).

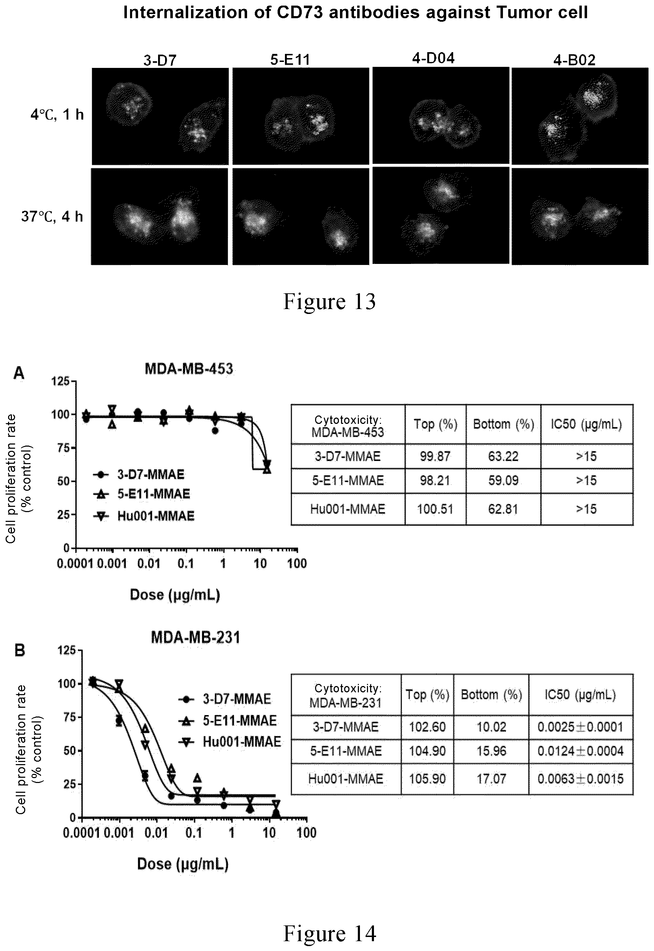

The results are shown in Figure 13. 3-D7, 5-E11, 4-D04, and 4-B02 can be rapidly and significantly internalized into lysosomes by MDA-MB-231 cells. This result shows that the antibodies of the present invention are suitable for preparing NDC, suggesting that CD73-NDC will have good NDC drug properties and the prospect of being used as broad-spectrum and highly specific targeting CD73-positive tumor therapeutic drugs.

### Example 9 Preparation of 3-D7-vc-MMAE, 5-E11-vc-MMAE, and 4-D01-vc-MMAE

### (I) Preparation of conjugate 3-D7-vc-MMAE

The 3-D07 protein stock solution was diluted to 5 mg/mL with 50 mM potassium dihydrogen phosphate-sodium hydroxide (KH₂PO₄-NaOH)/150 mM sodium chloride (NaCl)/1 mM diethyltriaminepentaacetic acid (DTPA) and reaction buffer of pH 7.4. Tris(2-carboxyethyl)phosphine hydrochloride (TCEP) with a 3-4 times excess molar ratio was added. The reaction solution was stirred at 25 °C for 2.5 hours.

The above reaction solution was cooled to 0-10 °C, and an appropriate amount of diethylacetamide (DMA) was added without purification, and then a 6 times excess molar ratio of mc-vc-MMAE (10 mg/ml pre-dissolved in DMA) was added to ensure that the volume proportion of DMA in the reaction system was no more than 10%, and the coupling was carried out by being stirred at 10-25 °C for 2 hours.

The coupling reaction mixture was purified by histidine-acetate/sucrose gel filtration at pH 6.0 using a desalting column, and the peak samples were collected according to the UV280 absorbance value. The samples were then sterilized through a 0.22 µm pore size filter and stored at -80 °C.

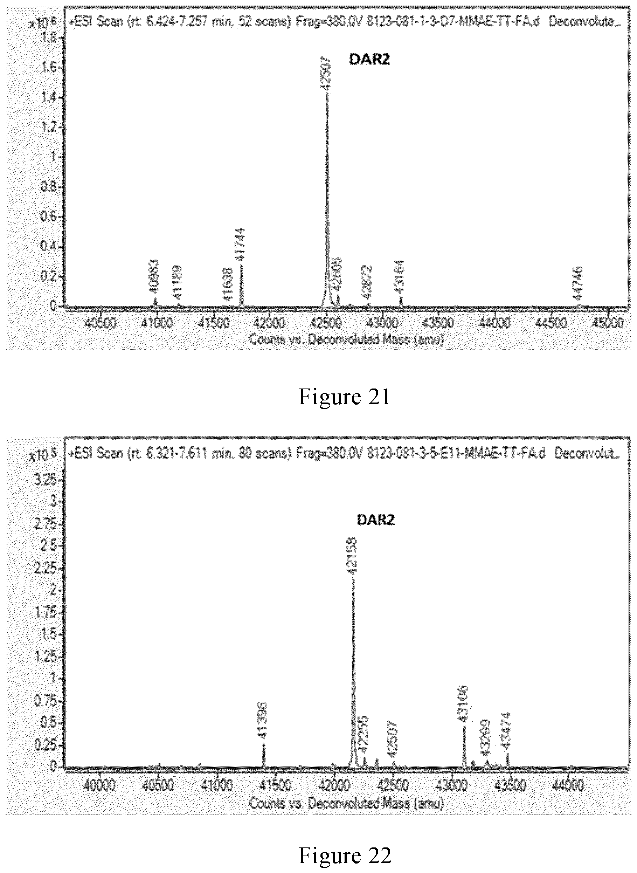

The exact drug loading was calculated to be approximately 2 by mass spectrometry analysis of the difference between the mass number and the theoretical molecular weight of the antibody conjugate 3-D7-vc-MMAE (Figure. 21).

### (II) Preparation of conjugate 5-E 11-vc-MMAE

The 5-E11 protein stock solution was diluted to 5 mg/mL with 50 mM potassium dihydrogen phosphate-sodium hydroxide (KH₂PO₄-NaOH)/150 mM sodium chloride (NaCl)/1 mM diethyltriaminepentaacetic acid (DTPA) and reaction buffer of pH 7.4. Tris(2-carboxyethyl)phosphine hydrochloride (TCEP) with a 3-4 times excess molar ratio was added. The reaction solution was stirred at 25 °C for 2.5 hours.

The above reaction solution was cooled to 0-10 °C, and an appropriate amount of diethylacetamide (DMA) was added without purification, and then a 6 times excess molar ratio of mc-vc-MMAE (10 mg/ml pre-dissolved in DMA) was added to ensure that the volume proportion of DMA in the reaction system was no more than 10%, and the coupling was carried out by being stirred at 10-25 °C for 2 hours.

The coupling reaction mixture was purified by histidine-acetate/sucrose gel filtration at pH 6.0 using a desalting column, and the peak samples were collected according to the UV280 absorbance value. The samples were then sterilized through a 0.22 µm pore size filter and stored at -80 °C.

The exact drug loading was calculated to be approximately 2 by mass spectrometry analysis of the difference between the mass number and the theoretical molecular weight of the antibody conjugate 5-E11-vc-MMAE (Figure 22).

### (III) Preparation of conjugate 4-D04-vc-MMAE

The 4-D04 protein stock solution was diluted to 5 mg/mL with 50 mM potassium dihydrogen phosphate-sodium hydroxide (KH₂PO₄-NaOH)/150 mM sodium chloride (NaCl)/1 mM diethyltriaminepentaacetic acid (DTPA) and reaction buffer of pH 7.4. Tris(2-carboxyethyl)phosphine hydrochloride (TCEP) with a 3-4 times excess molar ratio was added. The reaction solution was stirred at 25 °C for 2.5 hours.

The above reaction solution was cooled to 0-10 °C, and an appropriate amount of diethylacetamide (DMA) was added without purification, and then a 6 times excess molar ratio of mc-vc-MMAE (10 mg/ml pre-dissolved in DMA) was added to ensure that the volume proportion of DMA in the reaction system was no more than 10%, and the coupling was carried out by being stirred at 10-25 °C for 2 hours.

The coupling reaction mixture was purified by histidine-acetate/sucrose gel filtration at pH 6.0 using a desalting column, and the peak samples were collected according to the UV280 absorbance value. The samples were then sterilized through a 0.22 µm pore size filter and stored at -80 °C.

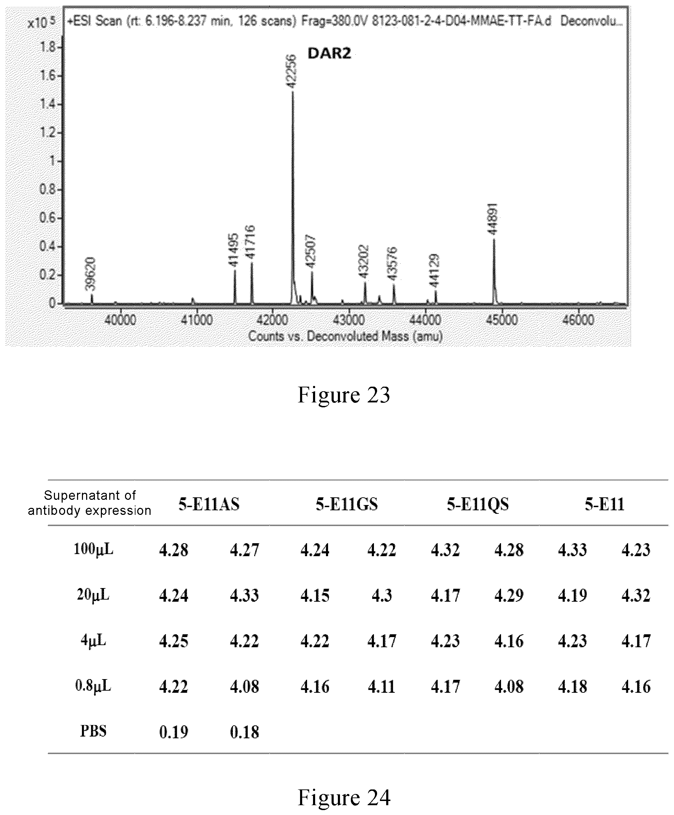

The exact drug loading was calculated to be approximately 2 by mass spectrometry analysis of the difference between the mass number and the theoretical molecular weight of the antibody conjugate 4-D04-vc-MMAE (Figure 23).

### Example 10 In vitro anti-tumor activity of CD73-NDC against tumor cells with CD73 high expression

The cell lines used in the example were purchased from the American Type Culture Collection (ATCC), the Cell Bank of the Chinese Academy of Sciences, and Nanjing Kebai Biotechnology Co., Ltd., and were cultured according to the corresponding instructions, including: MDA-MB-453, MDA-MB-231, PC9, NCI-H1975, NCI-H441, and SW1573. The above cells in logarithmic growth phase were seeded into 96-well cell culture plates 150 µL/well at a density of 200-2000 cells per well (depending on the growth rate of different cells), and cultured at 37°C, 5% CO₂ for about 5 hours. Then, different concentrations of CD73-NDCs (15 µg/mL to 0.00019 µg/mL) were added, 2-4 replicate wells were set for each drug concentration, and corresponding vehicle control and blank control wells were set. After 5-9 days of reaction (depending on the cell growth rate, to ensure a sufficient times of cell divisions), the culture medium was poured off, and MTS reaction solution (purchased from Promega, cat# G3581) was added 100 µL/well, and reacted at 37°C to the expected gradation of color. The cell viability (OD490nm) of each group was measured, and the cell survival rate was calculated according to the following formula: survival rate = (OD_{administration}-OD_{blank})/(OD_{control}-OD_{blank})×100%. The above data were analyzed by GraphPad Prism 8 software, and the IC₅₀ values of the above CD73 antibody-drug conjugates on different cell lines were calculated respectively.

The *in vitro* antitumor activity results of two preferred CD73-NDCs: 3-D7-MMAE, 5-E11-MMAE and CD73-ADC Hu001-MMAE as a control are shown in Figures 14 to 16, respectively.

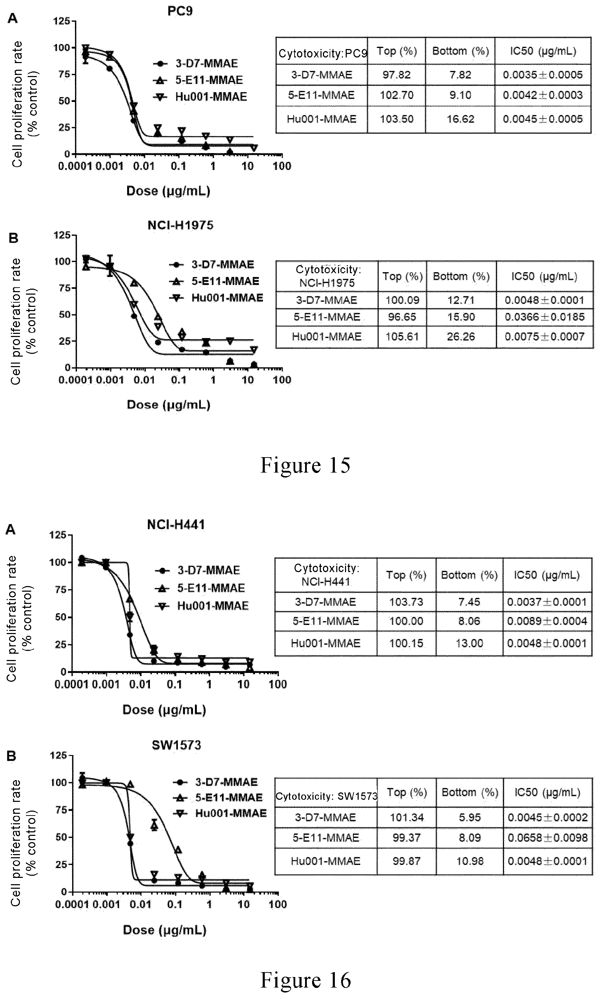

As shown in Figure 14A, CD73-NDCs had no obvious inhibitory effect on the proliferation of MDA-MB-453 cells with CD73 low expression, but showed strong inhibitory effects on MDA-MB-231 (Figure 14B), PC9 (Figure 15A), NCI-H1975 (Figure 15B), NCI-H441 (Figure 16A), and SW1573 (Figure 16B) cells with CD73 medium or high expression.

In general, the cytotoxicity (IC50 value) of CD73-NDC indicates that the cytotoxic activity of CD73-NDC is directly related to the CD73 expression level of the tested cells, and therefore it is determined as CD73 target-specific cytotoxicity. The IC50 values of some cell proliferation inhibition tests are summarized in Table-3.

**Table-3: In vitro antitumor activity of CD73 nanobody-drug conjugates**

| Cell Line | Cell proliferation inhibition rate IC50 ± SD (µg/mL) | | |
|---|---|---|---|
| | 3-D7-MMAE | 5-E11-MMAE | Hu001-MMAE |
| MDA-MB-453 | >15 | >15 | >15 |
| MDA-MB-231 | 0.0025 ± 0.0001 | 0.0124 ± 0.0004 | 0.0063 ± 0.0015 |
| PC9 | 0.0035 ± 0.0005 | 0.0042 ± 0.0003 | 0.0045 ± 0.0005 |
| NCI-H1975 | 0.0048 ± 0.0001 | 0.0366 ± 0.0185 | 0.0075 ± 0.0007 |
| NCI-H441 | 0.0037 ± 0.0001 | 0.0089 ± 0.0004 | 0.0048 ± 0.0001 |
| SW1573 | 0.0045 ± 0.0002 | 0.0658 ± 0.0098 | 0.0048 ± 0.0001 |

### Example 11 Protective effect of CD73-NDCs on T lymphocyte proliferation and IFN-γ expression

Human peripheral blood mononuclear cell (PBMC) cryogenic vials were provided by Jiangsu Xidier Biotechnology Co., Ltd. Firstly, PBMCs were revived and cultured for 3-4 days in a culture medium containing 500 ng/mL CD3/CD28 antibodies and 100 IU/mL IL-2, and then CD3-positive T lymphocytes were sorted out using a sorting kit (supplier: Stemcell, Cat#1795), and then fluorescently labeled. The cell suspension (final concentration of 2.5 µM) was added with pre-prepared fluorescent dye CFSE (carboxyfluorescein succinimidyl ester), incubated at 37°C for 5 min, and then washed 3 times with PBS before being used in the experiment.

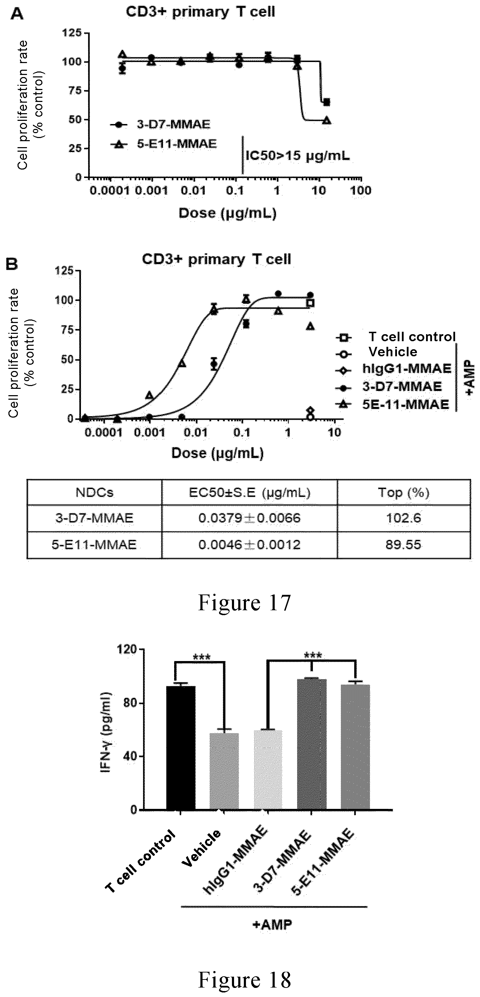

First the effect of different concentrations of CD73-NDC on the T cell proliferation curve was observed. CFSE-labeled T cells were plated in 96-well plates (2x10⁴ cells/well), and CD73-NDC (final concentration 15µg/mL to 0.000192µg/mL, n=2) or vehicle control was added to each well. After cultured for 5 days, the number of live cells was measured by FACS, and the IC₅₀ value was calculated based on a dose curve. As shown in Figure 17A, 3-D7-MMAE and 5-E11-MMAE did not show obvious toxic side effects in the tested concentration range (IC₅₀ >15 µg/mL).

In another set of experiments, CFSE-labeled T cells were plated in 96-well plates (2x10⁴ cells/well). 50 µL of gradient diluted NDC (final concentration ranged from 3 µg/mL to 0.00019 µg/mL, n=2) was added to each well, and 50 µL of AMP (final concentration of 0.25 mM) was added, mixed, and cultured for 4-5 days before collecting the culture supernatant. The number of cells in a fixed volume was measured and counted using a flow cytometer (FACS), and the cell proliferation curve was drawn using Flowjo software and the EC50 value was calculated. The results are shown in Figure 17B. 3-D7-MMAE and 5-E11-MMAE have a significant proliferation protective effect on human T lymphocytes and can effectively reverse the proliferation inhibitory effect of AMP on T lymphocytes, with EC₅₀ values of 0.0379 ± 0.0066 µg/mL and 0.0046 ± 0.0012 µg/mL, respectively.

50 µL/well of T cell culture supernatant was taken to detect IFN-γ protein concentration using ELISA kit (Sinobiological, Cat#KIT11725A) and by referring to the technical operation steps provided by the kit. The results are shown in Figure 18. 3-D7-MMAE and 5-E11-MMAE can effectively reverse the inhibitory effect of AMP on T cell expression/secretion of IFN-γ.

The results of this example show that CD73-NDC not only has no toxic effect on T lymphocytes, but also can effectively reverse the inhibitory effect of AMP/Ado on the proliferation of T lymphocytes.

### Example 12 Batch expression, production and purification of CD73 nanobodies and fusion proteins

The variable region sequences of 3-D7 and 5-E11 nanobodies were cloned into hIgG1 expression vectors containing different designed Fc fragments (3-D7-FC1, 3-D7-FC2, 3-D7-FC3, 5-E11-FC1, 5-E11-FC2, 5-E11-FC3), or recombinant/fusion protein expression vectors (3-D7-HLE, 3-D7-ABD, 5-E11-ABD), and then transiently transfected into HEK293 cells and suspended and cultured in serum-free medium for 6 days. The culture supernatant was collected for purification, protein quantification and SDS-PAGE detection.

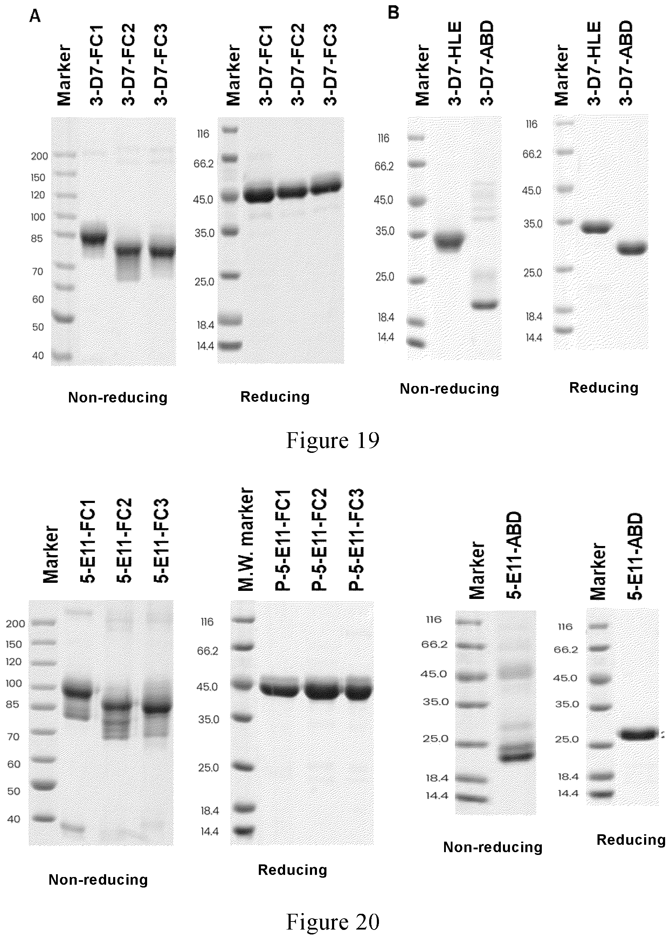

As shown in Figures 19 and 20, the chimeric nanobodies and recombinant fusion proteins derived from both 3-D7 and 5-E11 can be expressed, produced and purified with high efficiency.

### Example 13 Preparation and activity detection of CD73 nanobody 5-E11 derived antibodies

Due to the undesirable amino acid sequence (hot spot) in CDR2 of the original sequence of 5-E11 VH (SEQ ID NO. 8), it was mutated and modified to obtain 5-E11 derived antibodies 5-E11AS, 5-E11GS, 5-E11QS, and 5-E11ASCS, and the corresponding sequences are SEQ ID NO. 46, SEQ ID NO. 47, SEQ ID NO. 48, and SEQ ID NO. 49.

CD73 antigen ELISA binding affinity test was performed according to the method of Example 3. The results are shown in Figures 24. 5-E11AS, 5-E11GS, and 5-E11QS have comparable ELISA binding affinity to the original antibody 5-E11.

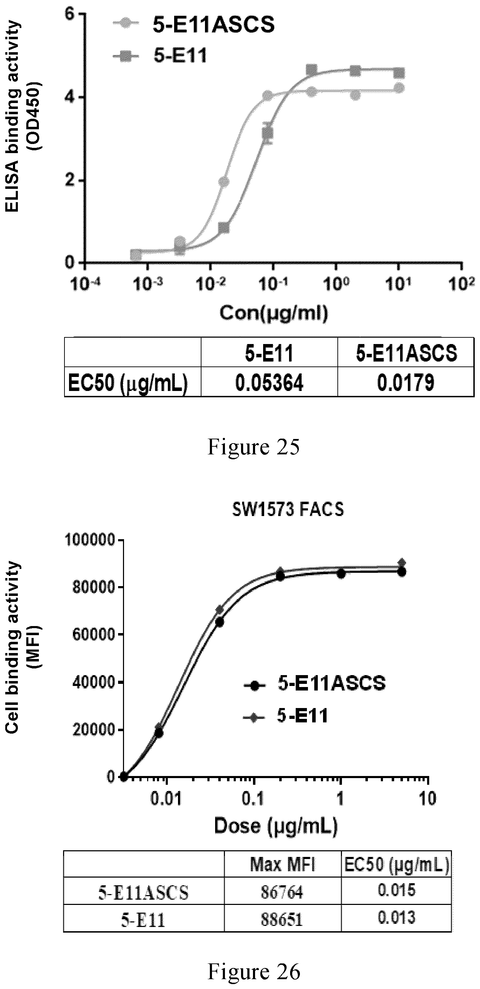

Based on the above mutants, 5-E11ASCS was designed and prepared, and the CD73 antigen ELISA binding affinity test was performed according to the method of Example 3. The results are shown in Figure 25. 5-E11ASCS has comparable binding affinity to the original antibody 5-E11, and the EC50 values are 0.018 µg/mL and 0.05 µg/mL, respectively.

The binding affinity of antibodies to CD73 on the surface of SW1573 cells was determined according to the method of Example 5. The results are shown in Figures 26. 5-E11ASCS and the original antibody 5-E11 have comparable cell FACS binding affinity, with EC₅₀ of 0.015 µg/mL and 0.013 µg/mL, respectively.

### Example 14 Humanization modification and activity detection of nanobody 3-D7

Based on the original antibody sequence information, the homology model of the antibody was obtained by modeling, and the framework amino acids within 5Å of CDRs were analyzed. These amino acid sites usually affect the conformation or antigen binding activity of CDRs. The humanized germline was obtained by IMGT analysis. After the selected humanized germline framework was spliced with the antibody CDRs, the framework region sequences of the designed humanized antibody and the original antibody were compared. By analyzing the homology modeling results of the parental antibody, amino acids similar to the surface residues of human antibodies were selected for replacement while maintaining the antibody activity and reducing heterology, thereby obtaining humanized antibodies 3D7-HM7, 3D7-HM8, 3D7HM9, and the corresponding sequences are SEQ ID NO.50, SEQ ID NO.51, and SEQ ID NO.52.

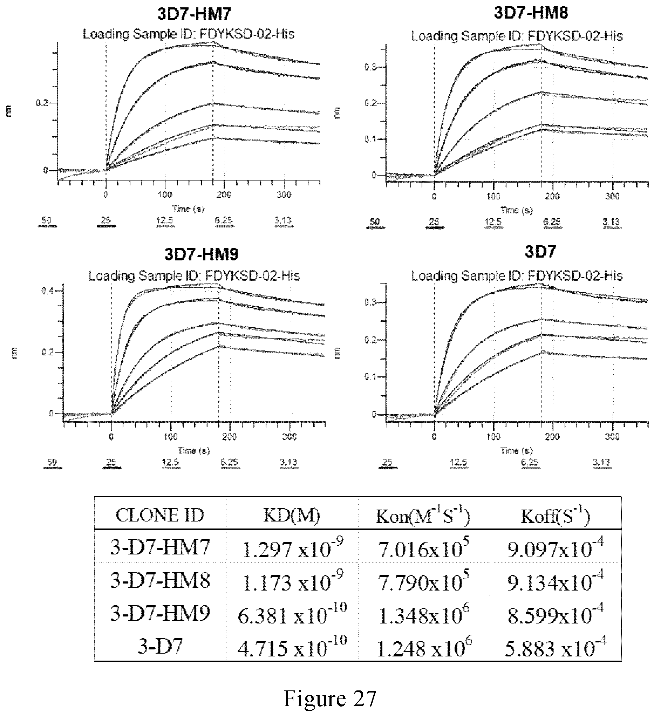

The affinity of the humanized antibody to the antigen was detected by the surface plasmon resonance (SPR) assay method in Example 2. The results are shown in Figure 27. 3D7-HM7, 3D7-HM8, 3D7-HM9 and the original antibody 3-D7 have extremely similar antigen binding affinity, and the binding constants are 1.297 nM, 1.173 nM, 0.638 nM, and 0.472 nM, respectively.

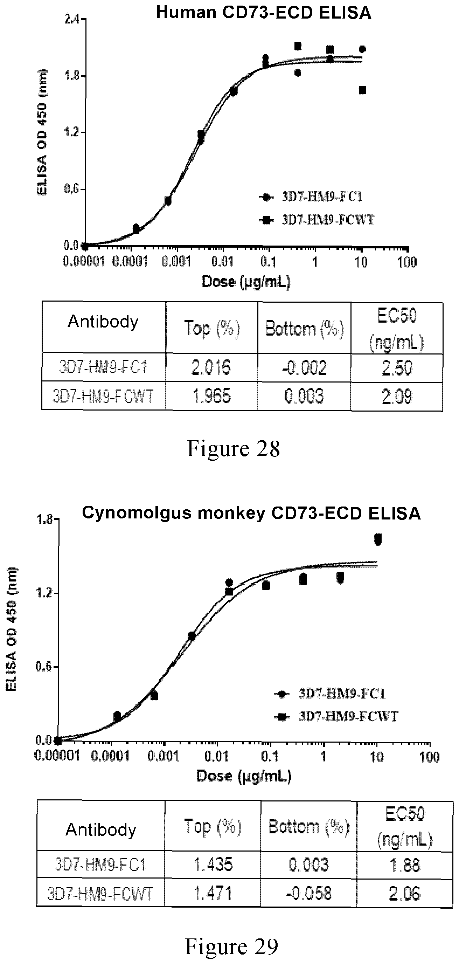

The affinity of the humanized antibody to human and cynomolgus CD73-ECD was detected according to the ELISA determination method of Example 3. The results are shown in Figure 28. The ELISA binding EC₅₀ of 3D7-HM9-FC1 and 3D7-HM9-FCWT to human CD73-ECD is 2.5 ng/mL and 2.09 ng/mL. The results are shown in Figure 29. The ELISA binding EC₅₀ of 3D7-HM9-FC1 and 3D7-HM9-FCWT to cynomolgus CD73-ECD is 1.88 ng/mL and 2.06 ng/mL.

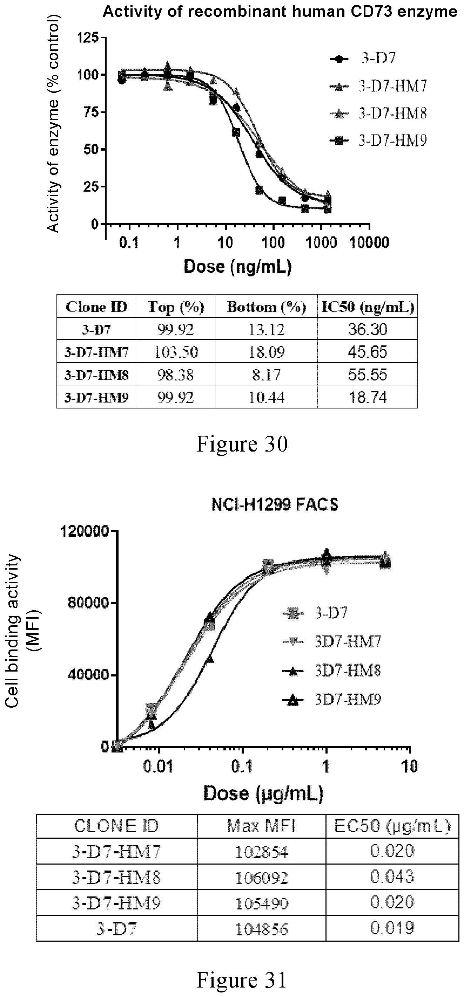

The inhibitory activity of the humanized antibody was detected according to the determination method of the catalytic function activity of the recombinant human CD73 enzyme in Example 4. The results are shown in Figure 30. 3D7-HM7, 3D7-HM8, 3D7-HM9 and the original antibody 3-D7 have extremely similar CD73 enzyme function inhibitory activity, with IC₅₀ of 45.65 ng/mL, 55.55 ng/mL, 18.74 ng/mL, 36.3 ng/mL, respectively.

The binding affinity of the humanized antibodies to NCI-H1299 cells was detected according to the method for determining the binding affinity of CD73 on the surface of tumor cells in Example 5. The results are shown in Figure 31. 3-D7-HM7, 3-D7-HM8, 3-D7-HM9 and the original antibody 3-D7 have extremely similar tumor cell binding affinity to, with EC₅₀ of 0.02 µg/mL, 0.043 µg/mL, 0.02 µg/mL, and 0.019 µg/mL, respectively.

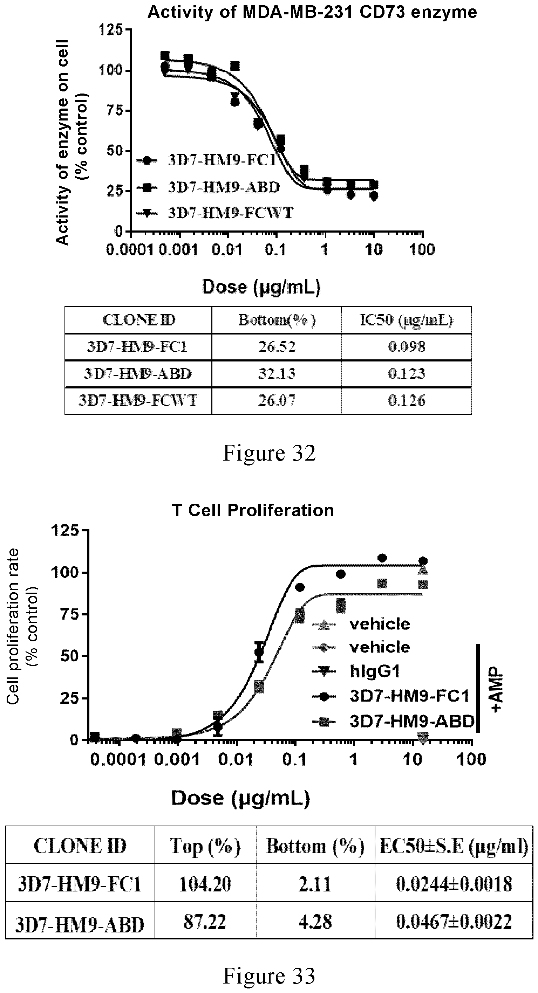

The inhibitory activity of the humanized antibody was detected according to the method for determining the catalytic function of CD73 enzyme on the surface of tumor cells in Example 6. The results are shown in Figure 32. 3D7-HM9-FC1, 3D7-HM9-ABD, 3D7-HM9-FCWT and the original antibody 3-D7 have extremely similar inhibitory activities, with IC₅₀ of 0.098 µg/mL, 0.123 µg/mL, and 0.126 µg/mL, respectively.

According to T lymphocyte proliferation method in Example 7, CFSE-labeled T cells were plated in a 96-well plate (2x10⁴ cells/well), 50 µL of nanobodies diluted in a gradient manner were added to each well (final concentration 15 µg/mL to 0.00019 µg/mL, n=4), and 50 µL of adenosine monophosphate (AMP, final concentration of 0.25 mM) were added, mixed, and the cells were cultured for 4-5 days. The number of cells in a fixed volume was measured and counted using a flow cytometer (FACS).

The results are shown in Figure 33. 3D7-HM9-FC1 and 3D7-HM9-ABD can effectively reverse the inhibitory effect of AMP on T lymphocyte proliferation, with EC₅₀ of 0.024 µg/mL and 0.0467 µg/mL, respectively.

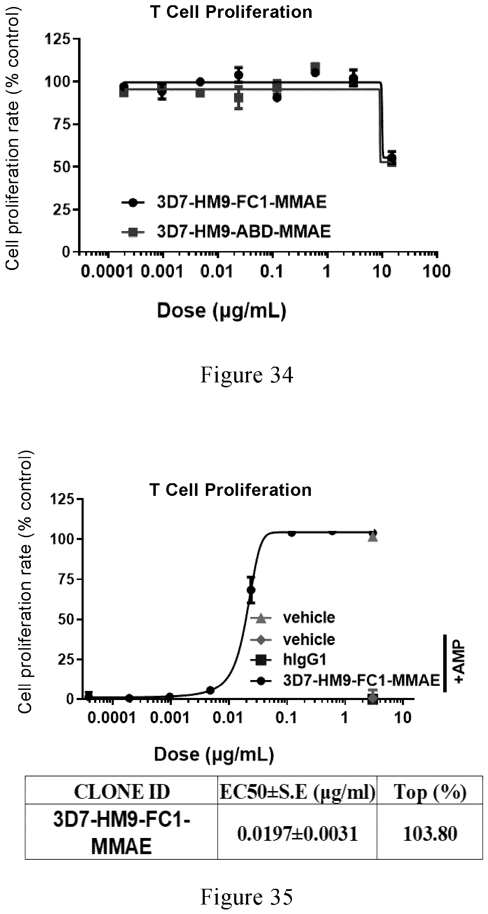

The effect of humanized NDC on T lymphocyte proliferation was detected according to Example 11. The results are shown in Figure 34. 3D7-HM9-FC1 and 3D7-HM9-ABD have no obvious toxic side effects on T lymphocyte proliferation, with an IC₅₀ > 10µg/mL.

In another set of tests, 0.25 mM AMP was added to the T lymphocyte proliferation incubation medium. The results are shown in Figure 35, showing that 3D7-HM9-FC1 can effectively reverse the inhibitory effect of AMP on T lymphocyte proliferation, with an EC₅₀ of 0.0197 µg/mL.

Vc-MMAE conjugates of humanized antibody fusion proteins 3D7-HM9-FC1 and 3D7-HM9-ABD were prepared according to Example 9. For naked antibodies and MMAE conjugates, the purity and coupling degree were tested by hydrophobic interaction chromatography (HIC), size exclusion chromatography (SEC), etc. It is expected that the desired DAR for a dimer conjugate formed by antibody and FC1 fusion protein is 4; the desired DAR for a conjugate with antibody-ABD structure as a monomer is 1.

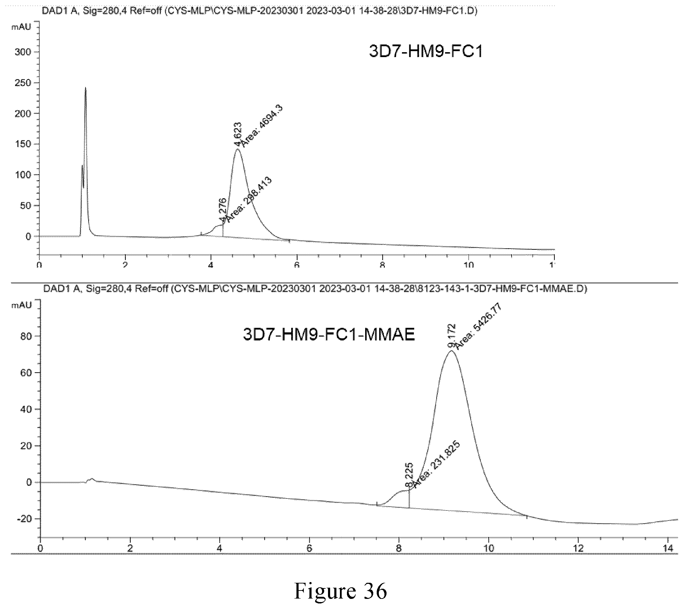

The results are shown in Figure 36. HIC analysis of the 3D7-HM9-FC1-MMAE conjugate showed a single main conjugate peak with an expected DAR value of 4.

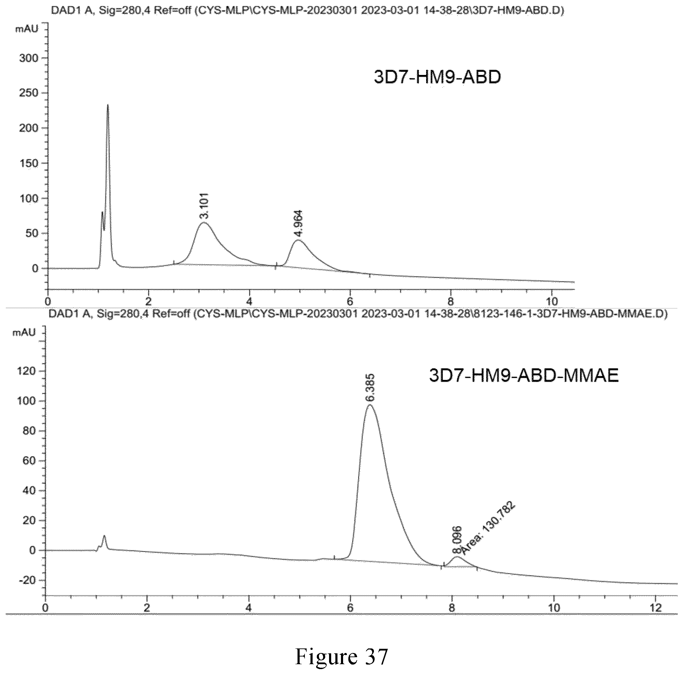

The results are shown in Figure 37. HIC detection analysis of the 3D7-HM9-ABD-MMAE conjugate showed a single conjugate main peak with an expected DAR value of 1.

The *in vitro* anti-tumor activity of humanized NDC on tumor cells with high expression of CD73 was detected according to Example 10. The cells tested included: lung cancer cell lines HCC44, PC9, H1975, SW1573, NCI-H441, and Calu-1, triple-negative breast cancer cell line MDA-MB-231, pancreatic cancer cell lines BXPC3 and HPAF-II, and brain glioma cell line U87MG. The above cells in the logarithmic growth phase were inoculated into 96-well cell culture plates at a density of 200-2000 cells per well (depending on the growth rate of different cells), 150 µL/well, 37°C, and treated with drugs for 5-9 days (depending on the cell growth rate, ensuring that the cells divide enough times). The culture medium was poured off, and the cell viability (OD490nm) of each group was measured.

The expected excellent *in vitro* antitumor activities of humanized NDC 3D7-HM9-FC1-MMAE, 3D7-HM9-FCWT-MMAE, and 3D7-HM9-ABD-MMAE against the above tumor cell lines were summarized in Table 4.

**Table-4: In vitro antitumor activity of humanized CD73 nanobody-drug conjugates**

| Cell Line | Cell proliferation inhibition rate IC50 ± SD (µg/mL) | | |
|---|---|---|---|
| | 3D7-HM9-FC1-MM AE | 3D7-HM9-FCWT-MM AE | 3D7-HM9-ABD-MM AE |
| HCC44 | 0.0087 ± 0.0002 | / | 1.3375 ± 0.3337 |
| PC9 | 0.0027 ± 0.0005 | 0.0037 ± 0.0007 | 0.4867 ± 0.0744 |
| NCI-H1975 | 0.0025 ± 0.0003 | / | 0.6482 ± 0.0855 |
| SW1573 | 0.0026 ± 0.0004 | 0.0033 ± 0.0006 | 0.8979 ± 0.0877 |
| NCI-H441 | 0.0030 ± 0.0004 | 0.0014 ± 0.0003 | 1.0680 ± 0.0455 |
| Calu-1 | 0.0029 ± 0.0005 | 0.0045 ± 0.0008 | 0.7992 ± 0.0676 |
| MDA-MB-2 31 | 0.0094 ± 0.0007 | 0.0780 ± 0.0054 | 2.0763 ± 0.8933 |
| BXPC3 | 0.0083 ± 0.0007 | 0.0124 ± 0.0011 | 1.2680 ± 0.0651 |
| HPAF-II | 0.0009 ± 0.0002 | 0.0020 ± 0.0003 | 0.3983 ± 0.0033 |
| U87MG | 0.0021 ± 0.0006 | 0.0005 ± 0.0001 | 0.4308 ± 0.0917 |

### Example 15 The killing effect of intermittent administration of CD73-NDC on tumor cells

1000 H1975 and SW1573 cells in the logarithmic growth phase were inoculated into 96-well cell culture plates, and cultured at 150 µL/well at 37 °C, 5% CO₂ for about 16 hours. 5µg/mL of hIgG1-MMAE, 3D7-FC1-MMAE, 5E11-FC1-MMAE and ADC Hu001-MMAE against the same target were added. The cells were treated for 24 h. The original culture medium was removed. The cells were washed gently with PBS once, and added with fresh complete culture medium, and continued to culture for 4 days without drugs. The culture medium was poured off, and MTS reaction solution was added 100 µL/well. The mixture was reacted at 37 °C until the expected gradation of color. The cell viability (OD₄₉₀ₙₘ) of each group was read with a microplate reader.

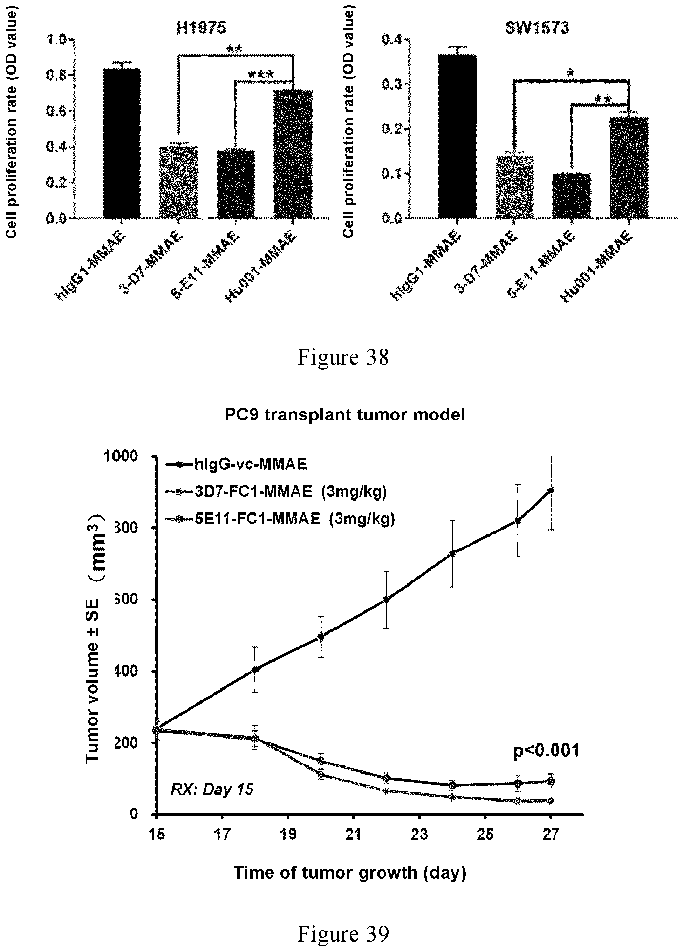

The results are shown in Figure 38. CD73-NDC showed a stronger killing effect on tumor cells by intermittent administration for one day compared with the ADC against the same target.

### Example 16 In vivo anti-tumor effect of CD73 nanobodies (subcutaneous tumor model)

4-5 weeks old nude mice were purchased and adaptively raised in an SPF animal laboratory for one week. PC9 cells in the logarithmic growth phase were digested and centrifuged in a 4 °C precooled centrifuge at 1000 rpm for 5 min. The supernatant was removed and the cells were resuspended in PBS according to the cell number. The cell density was adjusted to 25×10⁶/mL, and 200 µL of the cell suspension was inoculated subcutaneously in the ribs of the nude mice with 5×10⁶/tumor. The tumor growth was observed, and when the tumor grew to about 200 mm³, the mice were grouped according to the tumor volume and body weight. For PC9 transplantation tumor experiments (n=8), a single injection of 3 mg/kg of hIgG1-MMAE, 3D7-FC1-MMAE, and 5E11-FC1-MMAE was given through the tail vein on day 15 after tumor inoculation. The tumor growth and the state of the mice were observed regularly. The length and width of the tumor were measured twice a week, and the tumor volume was calculated according to the formula of tumor volume (mm³) = length × width × width ÷ 2. The tumor growth curve and mouse weight curve were drawn. At the end of the experiment, the mice were euthanized by carbon dioxide. The tumors were collected, the tumor weight was measured, and the tumor inhibition rate was calculated. The tumors were quickly frozen in liquid nitrogen and stored in a refrigerator at -80 °C.

The results are shown in Figure 39. 3D7-FC1-MMAE and 5E11-FC1-MMAE both showed excellent anti-tumor efficacy after a single administration of 3 mg/kg, and could result in significant tumor regression.

### Example 17 In vivo anti-tumor effect of CD73 nanobody (lung tumor orthotopic model)

4-5 weeks old nude mice were purchased and adaptively raised in an SPF animal laboratory for one week. HCC44-luc cells in the logarithmic growth phase were digested and centrifuged in a 4 °C precooled centrifuge at 1000 rpm for 5 min. The supernatant was removed and the cells were resuspended in PBS according to the cell number. The cell density was adjusted to 40×10⁶ cells/mL. An equal volume of high concentration matrix gel was added, i.e., cell suspension: matrix gel = 1:1. After the mice were anesthetized with Avertin (400 µL/20 g), a small incision was made on the right rib of the mouse near the armpit (three layers of skin and muscle in total), and the pink lungs were visible. The Y-shaped blood vessels on the skin were taken care to be avoided when opening the incision, and the needle can be inserted in the middle of the Y-shaped position. A line is drawn at the 3 mm position of the insulin needle, i.e., the insertion depth, and the cells were slowly pushed in. Each mouse was inoculated with 50 µL, a total of 1×10⁶ cells/mouse. After the injection was completed, it is stoped for 5-10 s, the needle was rotated out, and the wound was sutured.

In one set of experiments, the mice were imaged with a live imaging device on day 13 after modeling, and the mice were divided into groups according to the signal value. On day 13, 3 mg/kg of hIgG1-MMAE, 3D7-FC1-MMAE, and 5-E11-MMAE (n= 6) were injected into the tail vein once. On day 13 and day 20, 3 mg/kg of 3D7-ABD-MMAE (n=4) was injected into the tail vein twice. The fluorescence values were detected and recorded using a live imaging device on day 20, day 27, and day 34.

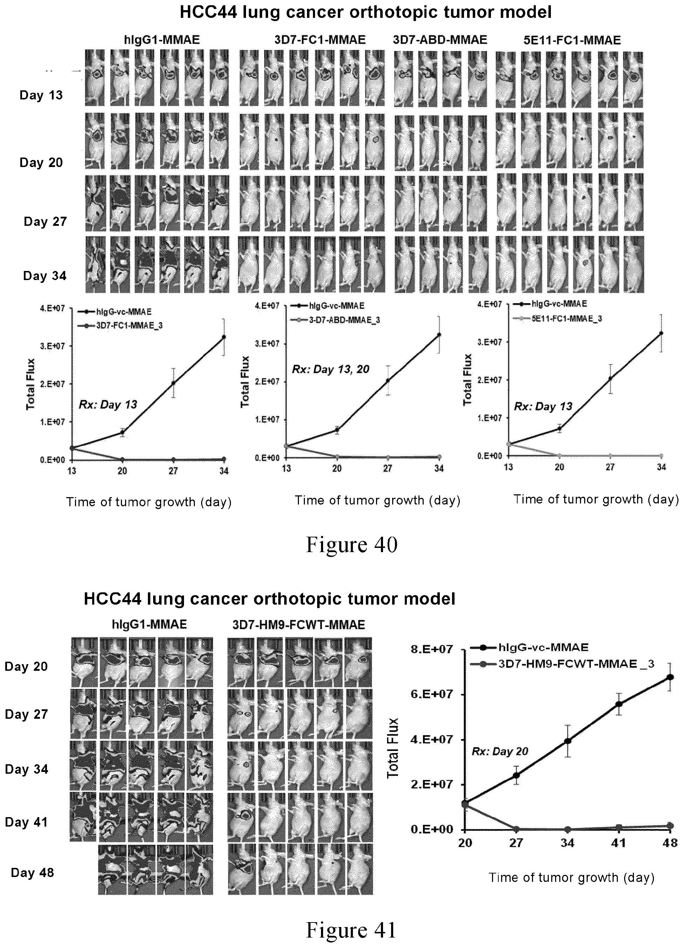

The results are shown in Figure 40. After a single administration of 3 mg/kg, 3D7-FC1-MMAE and 5E11-FC1-MMAE both showed excellent anti-tumor efficacy, resulting in tumor regression; 3D7-ABD-MMAE showed excellent anti-tumor efficacy after two doses, resulting in tumor regression.

In another group of experiments, 3 mg/kg of hIgG1-MMAE and 3D7-HM9-FCWT-MMAE were injected through the tail vein on day 20 after modeling, and the drug efficacy was observed according to the above steps.

The results are shown in Figure 41. 3D7-HM9-FCWT-MMAE exhibited excellent antitumor efficacy after a single administration of 3 mg/kg, resulting in regression of most tumors.

### Example 18 Isotope I¹²⁵-labeled antibody detection of brain entry

Firstly, H1975-1uc cells were inoculated into the brain. 1-2 weeks after inoculation, the luciferase substrate signal value was detected to determine that tumors were formed by tumor cells in the brain. The radioactive isotope I¹²⁵ labeled 3D7-FC1, 3D7-ABD, 5E11-ABD and IgG antibody Hu001 against the same target were injected into mice via the tail vein. The final dose for each mouse was 1 mg/kg, with a radioactivity of 300 µCi. Radioactivity was detected and the results were calculated at 1 hour and 3 hours after administration using a small animal SPECT/CT *in vivo* imaging system.

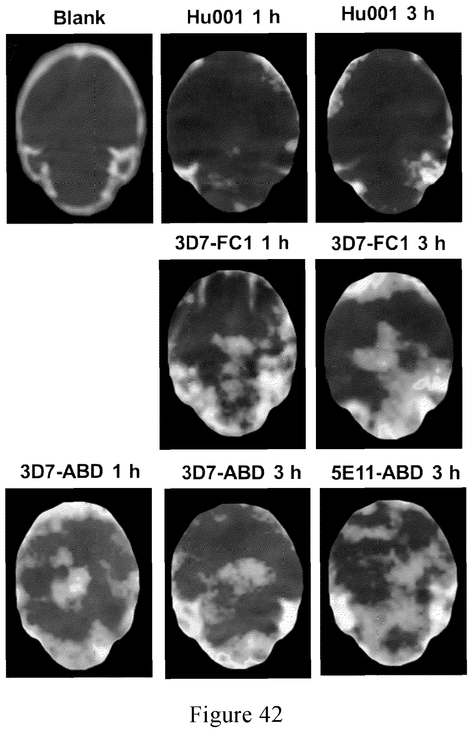

The results are shown in Figure 42. 1 hour and 3 hours after administration, the brains of mice injected with 3D7-FC1, 3D7-ABD, and 5E11-ABD showed very significant radioactive signals. The mice were observed 360 degrees using imaging software to confirm the signal values of the brain tumor inoculation site, while the corresponding Hu001 had a lower signal value in the brain.

### Example 19 In vivo anti-tumor effect of CD73 nanobody (intracranial model)

In one set of experiments, tumor fluorescence signal values were obtained by small animal *in vivo* imaging on day 12 after human lung cancer cell H1975-luc brain inoculation. Animals were divided into groups according to the fluorescence signal values, and 5 mg/kg of hIgG1-MMAE and 3D7-FC1-MMAE were respectively administered through the tail vein on day 13 (n=9). Tumor fluorescence signal values were detected by *in vivo* imaging every 7 days and curves were drawn.

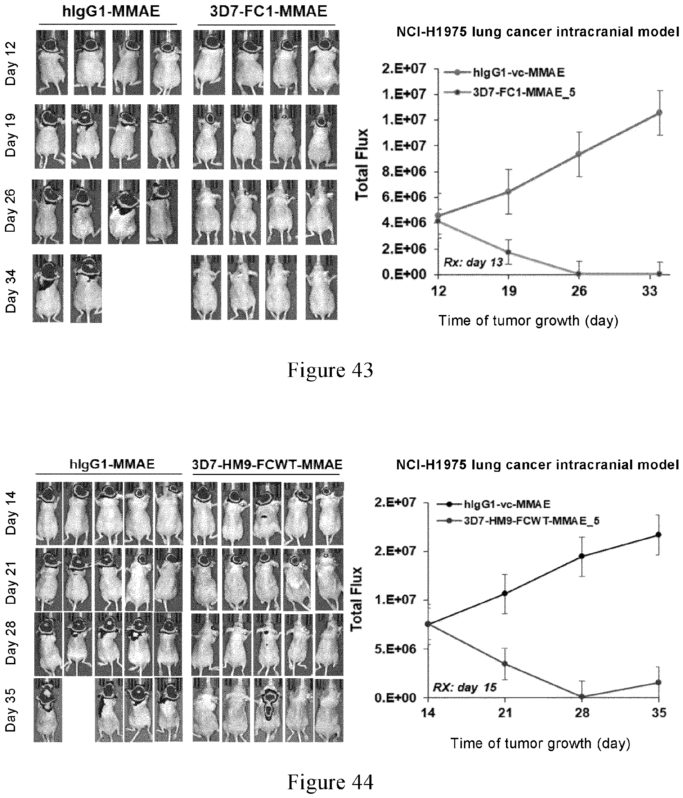

The results are shown in Figure 43. Compared with hIgG1-MMAE, after a single intravenous administration of 5 mg/kg 3D7-FC 1-MMAE, the growth of mouse lung cancer brain transplanted tumors was completely inhibited, and even the signal values of several tumors disappeared completely.

In another set of experiments, the H1975-luc brain inoculation was followed by intravital imaging on day 14 and the mice were divided into groups, and 5 mg/kg of hIgG1-MMAE and 3D7-HM9-FCWT-MMAE were administered via the tail vein on day 15 (n=5). Tumor fluorescence signal values were detected by intravital imaging every 7 days and curves were drawn.

The results are shown in Figure 44. Compared with hIgG1-MMAE, after a single intravenous administration of 5 mg/kg of 3D7-HM9-FCWT-MMAE, the growth of brain transplanted tumors in 4/5 mice was completely inhibited, and one mouse had tumor regression followed by recurrence.

In summary, the research in Examples related to CD73-NDC clearly shows that:
1. Both CD73-NDCs 3-D7-MMAE and 5-E11-MMAE have good CD73-specific tumor cell killing activity, which means they have a strong inhibitory effect on the proliferation of tumor cells with CD73 medium or high expression, but have no obvious toxicity to the proliferation of cells with CD73 low expression;
2. CD73-NDCs 3-D7-MMAE and 5-E11-MMAE have no obvious toxicity against the proliferation of normal human T lymphocytes;
3. CD73-NDCs 3-D7-MMAE and 5-E11-MMAE not only have no toxic effects on T lymphocytes, but can also effectively reverse the inhibitory effect of AMP/Ado on the proliferation of T lymphocytes; 4. In the intermittent administration model, NDCs showed stronger anti-tumor killing effect than the classic antibody-drug conjugate (ADC) against the same target.
5. The NDC of the present invention has extremely excellent *in vivo* anti-tumor activity and can completely inhibit tumor growth or cause tumor regression in mutiple tumor models after a single intravenous injection.
6. The nanobody and NDC of the present invention can penetrate the blood-brain barrier, have the characteristics of distribution in the brain, and show excellent therapeutic effects on intracranial tumors.
7. The humanized antibody and NDC of the present invention also exhibit the above-mentioned advantages.

### Sequences of nanobodies of the present invention

SEQ ID NO.1:3-D7 CDR1 GFLLDYYV
SEQ ID NO.2:3-D7 CDR2 ISGSDRST
SEQ ID NO.3:3-D7 CDR3 AADPSPVTVQAMCLPRFPVPY
SEQ ID NO.4:3-D7 VH 
QRQLVESGGGLVQPGGSLRLSCAASGFLLDYYVIGWFRQAPGKEREGV
SCISGSDRSTYYADSVKGRFTISRDNAKNTVYLQMNSLKPEDTAVYFCAADP
SPVTVQAMCLPRFPVPYWGQGTQVTVST
SEQ ID NO.5: 5-E11 CDR1 GFTLDYYN
SEQ ID NO.6: 5-E11 CDR2 SSNSGGST
SEQ ID NO.7: 5-E11 CDR3 AAYRGWHPSLDARVYDY
SEQ ID NO.8: 5-E11 VH 
ALQLVESGGGLVQPGGSLRLSCEVSGFTLDYYNIGWFRQAPGKEREG
VSCSSNSGGSTYYADSVKGRFTISRDNAKNTVYLQMNSLKPEDTGVYYCAA
YRGWHPSLDARVYDYWGQGTEVTVSS
SEQ ID NO.9: 4-D04 CDR1 GFTLDYYN
SEQ ID NO.10: 4-D04 CDR2 ISSSEGST
SEQ ID NO.11: 4-D04 CDR3 AADRYYYCSLHVSQYDY
SEQ ID NO.12: 4-D04 VH 
QLQLVESGGGLVQPGGSLRLSCAASGFTLDYYNIGWFRQAPGKEREG
VSCISSSEGSTYYADSVKGRFTISRDNAKNTVYLQMNSLKPEDTAVYYCAAD
RYYYCSLHVSQYDYWGQGTLVTVSS
SEQ ID NO.13: 4-B02 CDR1 GFPLDYYS
SEQ ID NO.14: 4-B02 CDR2 IGSSDGST
SEQ ID NO.15: 4-B02 CDR3 AALKYYYCSGHEAIYDY
SEQ ID NO.16: 4-B02 VH 
EVQLVESGGGLVQPGGSLRLSCAASGFPLDYYSIGWFRQAPGKEREG
VSCIGSSDGSTYYADSVKGRFTISRDNAENTVWLQMNSLKPEDTAVYYCAA
LKYYYCSGHEAIYDYWGQGTQVTVSG
SEQ ID NO.17: 1-E7 CDR1 GFTLDYYN
SEQ ID NO.18: 1-E7 CDR2 ISNSDGST
SEQ ID NO.19: 1-E7 CDR3 AAYRGWHPSLDARVYDY
SEQ ID NO.20: 1-E7 VH 
QVQLVESGGGLVQPGGSLRLSCAASGFTLDYYNIGWFRQAPGKERE
GVSCISNSDGSTYYADSVKGRFTISRDNAKNTVYLQMNSLKPEDTAVYYCA
AYRGWHPSLDARVYDYWGQGTQVTVSS
SEQ ID NO.21: 2-B10 CDR1 GFTLDDNA
SEQ ID NO.22: 2-B10 CDR2 MRTSDGDT
SEQ ID NO.23: 2-B10 CDR3 AAALGTYYSGFYYLAGDGMDY
SEQ ID NO.24: 2-B10 VH 
QLQLVESGGGLVQPGGSLRLSCAASGFTLDDNAIGWFRQAPGKEREG
VACMRTSDGDTYYADSVKGRFTISRDNAKNTMYLQMNSLKPEDTAVYYCA
AALGTYYSGFYYLAGDGMDYWGKGTLVTVSS
SEQ ID NO.25: 2-G9 CDR1 GFLLDYYT
SEQ ID NO.26: 2-G9 CDR2 ISSSDSSP
SEQ ID NO.27: 2-G9 CDR3 ALRANVYDY
SEQ ID NO.28: 2-G9 VH 
QLQLVESGGGLVQPGGSLRLSCAASGFLLDYYTIGWFRQAPGKELEG
VSCISSSDSSPYDADSVKGRFTMSRDNANNTVYLQMNSLKPEDTAVYYCAA
QRYYYCALRANVYDYWGQGTQVTVST
SEQ ID NO.29: 3-A3 CDR1 GFPLDYYT
SEQ ID NO.30: 3-A3 CDR2 ISSSDSSP
SEQ ID NO.31: 3-A3 CDR3 SLRANVYDY
SEQ ID NO.32: 3-A3 VH 
QVQLVESGGGSVQPGGSLRLSCAASGFPLDYYTIGWFRQAPGKELEG
VSCISSSDSSPYDADSVKGRFTMSRDNANNTVYLQMNSLKPEDTAVYYCAA
QRYYYCSLRANVYDYWGQGTQVTVSS
SEQ ID NO.33: 3-F1 CDR1 GFTLDYYT
SEQ ID NO.34: 3-F1 CDR2 IRSSDSSP
SEQ ID NO.35: 3-F1 CDR3 SLTANVYDY
SEQ ID NO.36: 3-F1 VH 
QVQLVESGVSLVQPGGSLRLSCAASGFTLDYYTIGWFRQAPGKELEG
ISCIRSSDSSPYDADSVKDRFTMSRDNANNTVYLQMNSLKPEDTADYYCAAQ
RYYYCSLTANVYDYWGQGTQVTVSS
SEQ ID NO.37: 3-D7-FC1 
QRQLVESGGGLVQPGGSLRLSCAASGFLLDYYVIGWFRQAPGKEREG
VSCISGSDRSTYYADSVKGRFTISRDNAKNTVYLQMNSLKPEDTAVYFCAAD
PSPVTVQAMCLPRFPVPYWGQGTQVTVSTEPKSSDKTHTCPPCPAPEFEGGP
SVFLFPPKPKDTLMISRTPEVTCVVVDVSHEDPEVKFNWYVDGVEVHNAKT
KPREEQYNSTYRVVSVLTVLHQDWLNGKEYKCKVSNKALPASIEKTISKAK
GQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLS
PGK
SEQ ID NO.38: 3-D7-FC2 
QRQLVESGGGLVQPGGSLRLSCAASGFLLDYYVIGWFRQAPGKEREG
VSCISGSDRSTYYADSVKGRFTISRDNAKNTVYLQMNSLKPEDTAVYFCAAD
PSPVTVQAMCLPRFPVPYWGQGTQVTVSTGGGGSGGGGSGGGGSPCPAPEF
EGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPASIEKTISK
AKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSL
SLSPGKC
SEQ ID NO.39: 3-D7-FC3 
QRQLVESGGGLVQPGGSLRLSCAASGFLLDYYVIGWFRQAPGKEREG
VSCISGSDRSTYYADSVKGRFTISRDNAKNTVYLQMNSLKPEDTAVYFCAAD
PSPVTVQAMCLPRFPVPYWGQGTQVTVSTEPKSSDKTHTCPPCPAPEFEGGP
SVFLFPPKPKDTLMISRTPEVTCVVVDVSHEDPEVKFNWYVDGVEVHNAKT
KPREEQYNSTYRVVSVLTVLHQDWLNGKEYKCKVSNKALPASIEKTISKAK
GQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLS
PGKC
SEQ ID NO.40: 5E11-FC1 
ALQLVESGGGLVQPGGSLRLSCEVSGFTLDYYNIGWFRQAPGKEREG
VSCSSNSGGSTYYADSVKGRFTISRDNAKNTVYLQMNSLKPEDTGVYYCAA
YRGWHPSLDARVYDYWGQGTEVTVSSEPKSSDKTHTCPPCPAPEFEGGPSVF
LFPPKPKDTLMISRTPEVTCVVVDVSHEDPEVKFNWYVDGVEVHNAKTKPR
EEQYNSTYRVVSVLTVLHQDWLNGKEYKCKVSNKALPASIEKTISKAKGQP
REPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPG
 K
SEQ ID NO.41: 5E11-FC2 
ALQLVESGGGLVQPGGSLRLSCEVSGFTLDYYNIGWFRQAPGKEREG
VSCSSNSGGSTYYADSVKGRFTISRDNAKNTVYLQMNSLKPEDTGVYYCAA
YRGWHPSLDARVYDYWGQGTEVTVSSGGGGSGGGGSGGGGSPCPAPEFEG
GPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPASIEKTISKA
KGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLS
LSPGKC
SEQ ID NO.42: 5E11-FC3 
ALQLVESGGGLVQPGGSLRLSCEVSGFTLDYYNIGWFRQAPGKEREG
VSCSSNSGGSTYYADSVKGRFTISRDNAKNTVYLQMNSLKPEDTGVYYCAA
YRGWHPSLDARVYDYWGQGTEVTVSSEPKSSDKTHTCPPCPAPEFEGGPSVF
LFPPKPKDTLMISRTPEVTCVVVDVSHEDPEVKFNWYVDGVEVHNAKTKPR
EEQYNSTYRVVSVLTVLHQDWLNGKEYKCKVSNKALPASIEKTISKAKGQP
REPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPG
KC
SEQ ID NO.43: 3-D7-HLE 
QRQLVESGGGLVQPGGSLRLSCAASGFLLDYYVIGWFRQAPGKEREG
VSCISGSDRSTYYADSVKGRFTISRDNAKNTVYLQMNSLKPEDTAVYFCAAD
PSPVTVQAMCLPRFPVPYWGQGTQVTVSTGGGGSGGGCGGGGSEVQLVES
GGGVVQPGGSLRLSCAASGLTFSSYAMGWFRQAPGKERERVVSISRGGGYT
YYADSVKGRFTISRDNSENTVYLQMNSLRPEDTALYYCAAARYWATGSEYE
FDYWGQGTLVTVSSAHHHHHHC
SEQ ID NO.44: 3-D7-ABD 
QRQLVESGGGLVQPGGSLRLSCAASGFLLDYYVIGWFRQAPGKEREG
VSCISGSDRSTYYADSVKGRFTISRDNAKNTVYLQMNSLKPEDTAVYFCAAD
PSPVTVQAMCLPRFPVPYWGQGTQVTVSTGGGGSGGGCGGGGSQHDEAV
DANSLAEAKVLANRELDKYGVSDYYKNLINNAKTVEGVKALIDEILAALPA
HHHHHHC
SEQ ID NO.45: 5-E11-ABD 
ALQLVESGGGLVQPGGSLRLSCEVSGFTLDYYNIGWFRQAPGKEREG
VSCSSNSGGSTYYADSVKGRFTISRDNAKNTVYLQMNSLKPEDTGVYYCAA
YRGWHPSLDARVYDYWGQGTEVTVSSGGGGSGGGCGGGGSQHDEAVDA
NSLAEAKVLANRELDKYGVSDYYKNLINNAKTVEGVKALIDEILAALPAHH
HHHC
SEQ ID NO.46: 5-E11AS VH 
ALQLVESGGGLVQPGGSLRLSCEVSGFTLDYYNIGWFRQAPGKEREGV
SCSSASGGSTYYADSVKGRFTISRDNAKNTVYLQMNSLKPEDTGVYYCAAY
RGWHPSLDARVYDYWGQGTEVTVSS
SEQ ID NO.47: 5-E11GS VH 
ALQLVESGGGLVQPGGSLRLSCEVSGFTLDYYNIGWFRQAPGKEREGV
SCSSGSGGSTYYADSVKGRFTISRDNAKNTVYLQMNSLKPEDTGVYYCAAY
RGWHPSLDARVYDYWGQGTEVTVSS
SEQ ID NO.48: 5-E11QS VH 
ALQLVESGGGLVQPGGSLRLSCEVSGFTLDYYNIGWFRQAPGKEREGV
SCSSQSGGSTYYADSVKGRFTISRDNAKNTVYLQMNSLKPEDTGVYYCAAY
RGWHPSLDARVYDYWGQGTEVTVSS
SEQ ID NO.49: 5-E11-ASCS VH 
ALQLVESGGGLVQPGGSLRLSCEVSGFTLDYYNIGWFRQAPGKEREGV
SSSSASGGSTYYADSVKGRFTISRDNAKNTVYLQMNSLKPEDTGVYYCAAY
RGWHPSLDARVYDYWGQGTEVTVSS
SEQ ID NO.50: 3D7-HM7 VH 
QRQLVESGGGLVQPGGSLRLSCAASGFLLDYYVIGWFRQAPGKEREGV
SCISGSDRSTYYADSVKGRFTISRDNSKNTVYLQMGSLRAEDTAVYYCAADP
SPVTVQAMCLPRFPVPYWGQGTLVTVSS
SEQ ID NO.51: 3D7-HM8 VH 
QRQLVESGGGLVQPGGSLRLSCAASGFLLDYYVIGWFRQAPGKEREGV
SCISGSDRSTYYADSVKGRFTISRDNSKNTVYLQMSSLRAEDTAVYYCAADP
SPVTVQAMCLPRFPVPYWGQGTLVTVSS
SEQ ID NO.52:3D7-HM9 VH 
QRQLVESGGGLVQPGGSLRLSCAASGFLLDYYVIGWFRQAPGKEREGV
SCISGSDRSTYYADSVKGRFTISRDNAKNTVYLQMSSLRAEDTAVYYCAADP
SPVTVQAMCLPRFPVPYWGKGTLVTVSS
SEQ ID NO.53:3D7-HM9-ABD 
QRQLVESGGGLVQPGGSLRLSCAASGFLLDYYVIGWFRQAPGKEREGV
SCISGSDRSTYYADSVKGRFTISRDNAKNTVYLQMSSLRAEDTAVYYCAADP
SPVTVQAMCLPRFPVPYWGKGTLVTVSSGGGGSGGGGSGGGGSQHDEAVD
ANSLAEAKVLANRELDKYGVSDYYKNLINNAKTVEGVKALIDEILAALPAA
ACAAAHHHHHH
SEQ ID NO.54 5-E11 AS CDR2 or 5-E11 ASCS CDR2: SSASGGST
SEQ ID NO.55 5-E11 GS CDR2 SSGSGGST
SEQ ID NO.56 5-E11 QS CDR2 SSQSGGST

All literatures mentioned in the present application are incorporated herein by reference, as though each one is individually incorporated by reference. In addition, it should also be understood that, after reading the above teachings of the present invention, those skilled in the art can make various changes or modifications, and these equivalents also fall in the scope of claims as defined in the appended claims.

## Claims

1. A nanobody targeting CD73, wherein the complementary determining region CDR of the VHH chain in the nanobody is one or more selected from the group consisting of:
(1) CDR1 as shown in SEQ ID NO.1,
CDR2 as shown in SEQ ID NO.2, and
CDR3 as shown in SEQ ID NO.3;
or,
(2) CDR1 as shown in SEQ ID NO.5,
CDR2 as shown in SEQ ID NO.6, and
CDR3 as shown in SEQ ID NO.7;
or,
(3) CDR1 as shown in SEQ ID NO.9,
CDR2 as shown in SEQ ID NO.10, and
CDR3 as shown in SEQ ID NO.11,
or,
(4) CDR1 as shown in SEQ ID NO.13,
CDR2 as shown in SEQ ID NO.14, and
CDR3 as shown in SEQ ID NO.15,
or,
(5) CDR1 as shown in SEQ ID NO.17,
CDR2 as shown in SEQ ID NO.18, and
CDR3 as shown in SEQ ID NO.19,
or,
(6) CDR1 as shown in SEQ ID NO.21,
CDR2 as shown in SEQ ID NO.22, and
CDR3 as shown in SEQ ID NO.23,
or,
(7) CDR1 as shown in SEQ ID NO.25,
CDR2 as shown in SEQ ID NO.26, and
CDR3 as shown in SEQ ID NO.27,
or,
(8) CDR1 as shown in SEQ ID NO.29,
CDR2 as shown in SEQ ID NO.30, and
CDR3 as shown in SEQ ID NO.31,
or,
(9) CDR1 as shown in SEQ ID NO.33,
CDR2 as shown in SEQ ID NO.34, and
CDR3 as shown in SEQ ID NO.35; or
(10) CDR1 as shown in SEQ ID NO.5,
CDR2 as shown in SEQ ID NO.54, and
CDR3 as shown in SEQ ID NO.7; or
(11) CDR1 as shown in SEQ ID NO.5,
CDR2 as shown in SEQ ID NO.55, and
CDR3 as shown in SEQ ID NO.7; or
(12) CDR1 as shown in SEQ ID NO.5,
CDR2 as shown in SEQ ID NO.56, and
CDR3 as shown in SEQ ID NO.7.

2. The nanobody according to claim 1, wherein the VHH chain of the nanobody further comprises a framework region (FR).

3. An antibody targeting CD73, wherein the antibody comprises one or more VHH chains of the nanobody targeting CD73 according to claim 2.

4. A multispecific antibody, wherein the multispecific antibody comprises: the nanobody targeting CD73 according to claim 1 or the antibody targeting CD73 according to claim 3.

5. A recombinant protein, wherein the recombinant protein comprises:
(i) the nanobody targeting CD73 according to claim 1, or the antibody targeting CD73 according to claim 3; and
(ii) an optional polypeptide molecule or fragment with therapeutic function; and/or
(iii) an optional functional domain for improvement of the physicochemical properties or druggability of the protein.

6. The recombinant protein according to claim 5, wherein the recombinant protein has the following elements from the N-terminus to C-terminus:
A-B;
wherein the element A is the nanobody targeting CD73; the element B is an Fc segment, an albumin binding domain (ABD) or an anti-albumin nanobody (HLE);
"-" represents a peptide bond or linker.

7. The recombinant protein according to claim 5, wherein the VHH chain of the nanobody targeting CD73 is selected from the group consisting of: the amino acid sequence shown in SEQ ID NO.4, SEQ ID NO.8, SEQ ID NO.12, SEQ ID NO.16, SEQ ID NO.20, SEQ ID NO.24, SEQ ID NO.28, SEQ ID NO.32, SEQ ID NO.36, SEQ ID NO.46, SEQ ID NO.47, SEQ ID NO.48, SEQ ID NO.49, SEQ ID NO.50, SEQ ID NO.51 or SEQ ID NO.52.

8. A CAR construct, wherein the antigen binding region of the CAR construct is the VHH chain of the nanobody according to claim 1.

9. A recombinant immune cell, wherein the immune cell expresses the exogenous CAR construct according to claim 8.

10. An immunoconjugate, wherein the immunoconjugate comprises:
(a) an antibody moiety, wherein the antibody moiety is the nanobody targeting CD73 according to claim 1 or the antibody targeting CD73 according to claim 3; and
(b) a coupling moiety coupled to the nanobody moiety, wherein the coupling moiety is selected from the group consisting of: a detectable marker, a drug, a toxin, a cytokine, an enzyme, a protein degrader, an oligonucleotide or a combination thereof.

11. The immunoconjugate according to claim 10, wherein the protein degrader is a degrader of a tumor-related protein selected from the group consisting of: EGFR, NF-κB, RIPK2, BCR-ABL, HER2, c-Met, TBK1, CDK, ALK, Akt, CK2, ERK1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3 FRS2, and RAS (e.g., KRAS, HRAS, and NRAS).

12. The immunoconjugate according to claim 10, wherein the immunoconjugate is an antibody-drug conjugate ADC as shown in the following molecular formula: 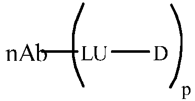 wherein:
nAb is the nanobody targeting CD73,
LU is a linker (also called a connector);
D is a drug;
and the subscript p is a value selected from 1-8.

13. The immunoconjugate according to claim 10, wherein LU is selected from maleimidocaproyl (MC), maleimide (MAL), succinimidyl 4-(N-maleimidomethyl) cyclohexane-1-carboxylate) (SMCC) linkers linked to the antibody moiety, and comprises one or more of valine-citrulline (VC), valine-alanine (VA), glycine-glycine-phenylalanine-glycine (GGFG), alanine-alanine-alanine (AAA), p-aminobenzyloxycarbonyl (PAB), and polyethylene glycol (PEG).

14. The immunoconjugate according to claim 10, wherein D is a compound with anti-tumor activity selected from the group consisting of:
(i) a tubulin inhibitor, such as maytansine derivative (DM1, DM4), monomethyl auristatin E (MMAE), monomethyl auristatin F (MMAF);
(ii) a toxin that acts on DNA, such as duocarmycin and pyrrolobenzodiazepine (PBD);
(iii) a topoisomerase inhibitor, camptothecin, SN38, Exitecan, Dxd.

15. A pharmaceutical composition, wherein the pharmaceutical composition comprises:
(i) the nanobody targeting CD73 according to claim 1, the antibody targeting CD73 according to claim 3, the multispecific antibody according to claim 4, the recombinant protein according to any one of claims 5 to 7, the recombinant immune cell according to claim 9, or the immunoconjugate according to any one of claims 10 to 14;
(ii) a pharmaceutically acceptable carrier.

16. Use of an active ingredient, wherein the active ingredient is selected from the group consisting of: the nanobody targeting CD73 according to claim 1, the antibody targeting CD73 according to claim 3, the multispecific antibody according to claim 4, the recombinant protein according to any one of claims 5-7, the recombinant immune cell according to claim 9, or the immunoconjugate according to any one of claims 10 to 14, or a combination thereof, wherein the active ingredient is used for (a) preparing a detection reagent, a detection plate or a kit; and/or (b) preparing a medicine for the prevention and/or treatment of a CD73-related disease.

17. A method (including diagnostic or non-diagnostic method) for detectionof CD73 in a sample *in vitro,* wherein the method comprises the steps of:
(1) contacting the sample with the nanobody targeting CD73 according to claim 1, the antibody targeting CD73 according to claim 3, or the immunoconjugate according to any one of claims 10-14 *in vitro;*
(2) detecting whether an antigen-antibody complex is formed, wherein the formation of the complex indicates the presence of CD73 in the sample.
